(19) **Europäisches Patentamt / European Patent Office / Office européen des brevets**

(11) **EP 2 844 144 B1**

(12) ## EUROPEAN PATENT SPECIFICATION

(45) Date of publication and mention
of the grant of the patent:
**23.11.2016 Bulletin 2016/47**

(21) Application number: **13784410.6**

(22) Date of filing: **01.05.2013**

(51) Int Cl.:
*A61B 5/085* (2006.01)    *A61B 5/087* (2006.01)
*A61M 16/00* (2006.01)    *G01H 11/08* (2006.01)
*H01L 41/09* (2006.01)    *A61B 5/00* (2006.01)
*A61B 5/097* (2006.01)    *A61M 16/08* (2006.01)
*A61M 16/04* (2006.01)    *A61M 15/00* (2006.01)

(86) International application number:
**PCT/CA2013/000433**

(87) International publication number:
**WO 2013/163740 (07.11.2013 Gazette 2013/45)**

(54) **PIEZOELECTRIC BEAM BENDING ACTUATED DEVICE FOR MEASURING RESPIRATORY SYSTEM IMPEDANCE**

PIEZOELEKTRISCHER STABFÖRMIGER BIEGEWANDLER ZUR MESSUNG EINER ATMUNGSSYSTEMIMPEDANZ

DISPOSITIF ACTIONNÉ PAR CINTRAGE DE FAISCEAU PIÉZOÉLECTRIQUE POUR MESURE D'IMPÉDANCE DE SYSTÈME RESPIRATOIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.05.2012 US 201261640797 P**

(43) Date of publication of application:
**11.03.2015 Bulletin 2015/11**

(73) Proprietor: **Dalhousie University**
**Halifax, Nova Scotia B3H 4H6 (CA)**

(72) Inventors:
• **MAKSYM, Geoffrey N.**
  **Dartmouth, Nova Scotia B2Y 2E9 (CA)**
• **POSADA, Lucas**
  **Montreal, QC**
  **H2S 1B4 (CA)**

• **HANAFIALAMDARI, Hamed**
  **Halifax, Nova Scotia**
  **B3H 3B8 (CA)**

(74) Representative: **Cohausz & Florack**
**Patent- & Rechtsanwälte**
**Partnerschaftsgesellschaft mbB**
**Bleichstraße 14**
**40211 Düsseldorf (DE)**

(56) References cited:
| JP-A- S60 187 799 | US-A- 6 074 350 |
| US-A1- 2007 006 924 | US-A1- 2011 092 840 |
| US-A1- 2011 228 968 | US-B2- 7 368 855 |

• KACZKA ET AL.: 'Oscillation mechanics of the respllatom svstem: applications to lung disease' CRIT. REV. BIONZED ENG. vol. 39, no. 4, 2011, pages 337 - 359, XP055157519

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD**

The present invention relates to an actuator according to claim 1. Such an actuator is for example known from JP S60 187799 A. Moreover, the present invention relates to a forced oscillation technique device comprising such an actuator with the features of claim 10. Finally, the present invention relates to a method for determining the respiratory impendance of a subject according to claim 13.

**BACKGROUND**

Forced oscillation technique (FOT) is a noninvasive method with which to measure respiratory mechanics. FOT techniques apply oscillating external pressure signals to a subject's normal breathing and measure the oscillatory respiratory flows arising from the oscillating external pressure.

FOT is a reliable method in the assessment of bronchial hyperresponsiveness in adults and children. Moreover, in contrast with spirometry where a deep inspiration is needed, forced oscillation technique does not modify the airway smooth muscle tone. Forced oscillation technique has been shown to be as sensitive as spirometry in detecting impairments of lung function due to smoking or exposure to occupational hazards. The FOT has the advantages that it can be performed on subjects that may not be compliant or in physiological states that cannot comply with conscious maneuvers. The FOT is particularly advantageous for the measurement of respiratory mechanics in infants and young children that would find difficulty in complying with traditional spirometry.

The increase in the prevalence of chronic respiratory diseases, such as asthma and chronic obstructive pulmonary disease (COPD) has resulted in a greater need for methods of assessing lung health. Along with increase in prevalence of respiratory diseases there has been a rise in health services provided at health care centers, at home or by telemedicine systems.

The FOT was designed to apply flow oscillations of varying frequencies at the airway opening during voluntary apnoea. The principle is that the forced oscillations at the airway opening are applied at frequencies greater than the respiration frequency and its harmonics, thus the pressure and flow registered by the FOT device are for the most part independent of the underlying respiratory pattern. This implies that the driving pressure at the forced oscillation frequency is the pressure attributable to the oscillations in the device since the activity of the muscle pump is negligible at such high frequency. The person's respiratory mechanics at the oscillation frequency then can be determined by the pressure and flow registered at the airway opening even though the recorded pressure and flow signals still contain both the inherent respiratory system pressure and flow and the superimposed forced oscillation signals.

Depending on its design, each type of FOT device is capable of generating a characteristic oscillation signal. These external forcing signals maybe mono-frequency, multi-frequency, and may also be applied either continuously (as in the FOT) or in a time-discrete manner (as in the IOS which uses impulses). In most FOT devices, the pressure oscillations or impulses are generated by a loudspeaker-in-box assembly which, by default, requires a speaker with a large membrane. As a test of pulmonary function, the FOT overcomes one of the key limitations of spirometry as it requires only passive cooperation; subjects breathe through a mouthpiece during the tests. In the FOT manoeuvre oscillatory pressure signals around 1 - 2 cmH$_2$O are superimposed on the spontaneous respiration of the subject, and respiratory system mechanical parameters are then estimated from the impedance of the respiratory system (Zrs) to the resulting flow oscillations.

The respiratory impedance (Zrs) that is measured is the spatial ratio of the Fourier Fast Transform (FFT) of the pressure (Prs) and flow (V'ao) measured at a person's airway opening (Equation 1).

$$Z(f) = \frac{Prs(f)}{V'ao(f)} \qquad (1)$$

Zrs is a complex quantity and consists of a real and an imaginary part (Equation 2). The real part describes the resistance of the respiratory system (Rrs) which is governed largely by the inner diameters of the airways while the imaginary part describes the reactance of the respiratory system (Xrs) which is governed largely by the elasticity of the lung and chest tissues and inertia of the oscillating air.

$$Zrs(f) = Rrs(f) + jXrs(f) \qquad (2)$$

Current FOT devices are generally based on large loudspeakers that are connected to the subject's airway opening via long tubing. Airflow is estimated or measured using pneumotachographs. These FOT devices are large and expensive. Reducing the cost and size of the device would aid in monitoring of respiratory mechanics in ambulatory and home care applications. Thus there is a need for FOT devices that are less expensive and more lightweight.

It is therefore an object of the present invention to provide an actuator, a corresponding forced oscillation technique device and a corresponding method with which the cost and weight can be reduced.

A device according to the prior art is known from US 2011/0228968.

## BRIEF DESCRIPTION OF THE DRAWINGS

The disclosure will now be described, by way of example, with reference to the accompanying drawings, in which:

FIG. 1A is a representative view of a single cantilever actuator of a forced oscillation technique device according to an embodiment.

FIG. 1B is a representative view of a multi cantilever actuator of a forced oscillation technique device according to an embodiment.

FIG. 1C is a representative view of a multi cantilever actuator of a forced oscillation technique device according to an embodiment.

FIG. 2 is a view of a forced oscillation technique impedance measuring device including the single cantilever actuator of FIG. 1A according to an embodiment.

FIG. 3A is a cross-sectional view according to line 3-3 of FIG. 2 according to an embodiment.

FIG. 3B is a cross-sectional view according to line 3-3 of FIG. 2 according to an embodiment.

FIGS. 4A-4C are partial perspective views of the forced oscillation technique impedance measuring device of FIG. 2 illustrating the single cantilever actuator arranged in forwardly, neutral and rearwardly orientations, respectively.

FIGS. 5A-5C are side views of FIGS. 4A-4C.

FIG. 6A is a front view of a forced oscillation technique impedance measuring device including the single cantilever actuator of FIG. 1A according to an embodiment.

FIG. 6B is a side view of the forced oscillation technique impedance measuring device of FIG. 6A.

FIG. 7A is a cross-sectional view according to line 7A-7A of FIG. 6A.

FIG. 7B is a cross-sectional view according to line 7B-7B of FIG. 6A.

FIG. 7C is a cross-sectional view according to line 7C-7C of FIG. 6B.

FIG. 8A is an enlarged view according to line 8A of FIG. 6A.

FIG. 8B is an enlarged view according to line 8B of FIG. 6B.

FIG. 9A is a side view of a forced oscillation technique impedance measuring device including the multi cantilever actuator of FIG. 1B according to an embodiment.

FIG. 9B is a partial perspective view of the forced oscillation technique impedance measuring device of FIG. 9A.

FIG. 10A is a side view of a forced oscillation technique impedance measuring device including the multi cantilever actuator of FIG. 1B according to an embodiment.

FIG. 10B is a perspective view of the forced oscillation technique impedance measuring device of FIG. 10A.

FIG. 11 is a perspective view of a forced oscillation technique impedance measuring device including the multi cantilever actuator of FIG. 1C according to an embodiment.

FIGS. 11A-11C are side views of the forced oscillation technique impedance measuring device of FIG. 11 illustrating the multi cantilever actuator of FIG. 1C arranged in forwardly, neutral and rearwardly orientations, respectively.

FIG. 12 is a partial perspective view of a forced oscillation technique impedance measuring device including the multi cantilever actuator of FIG. 1C according to an embodiment.

FIG. 13 is a partial perspective view of a forced oscillation technique impedance measuring device including the multi cantilever actuator of FIG. 1C according to an embodiment.

FIG. 14 is a partial perspective view of a forced oscillation technique impedance measuring device including the multi cantilever actuator of FIG. 1C according to an embodiment.

FIG. 15 is a partial perspective view of a forced oscillation technique impedance measuring device including the multi cantilever actuator of FIG. 1C according to an embodiment.

FIG. 16A is a side view of a forced oscillation technique impedance measuring device including the multi cantilever actuator of FIG. 1C according to an embodiment.

FIG. 16B is a front view of the forced oscillation technique impedance measuring device of FIG. 16A.

FIG. 17A is a cross-sectional view according to line 17A-17A of FIG. 16A.

FIG. 17B is a cross-sectional view according to line 17B-17B of FIG. 16B.

FIG. 18A is an enlarged view according to line 18A of FIG. 16A.

FIG. 18B is an enlarged view according to line 18B of FIG. 16B.

FIG. 19A is a schematic drawing illustrating the structural features of a monomorph piezoelectric element.

FIG. 19B is a schematic drawing illustrating the structural features of a bimorph piezoelectric element.

FIG. 20 is a line graph of various mesh surface areas and their effect on displacement amplitude versus frequency of waveform exposure using a single piezoelectric active material.

FIG. 21 is a line graph comparing mass of the top of a piezoelectric actuator and its resultant natural frequency resonance.

FIG. 22 is a plot of displacement amplitude and resultant frequency response for two disks scanned during a frequency sweep chirp input from 0 to 27 Hz.

FIG. 23 is a bar graph of resulting signal to noise ratios (SNR) computed from 10 tests of flow pressure and displacement using disks of the present invention.

FIG. 24 is a line graph of various mesh surface areas and their effect on displacement amplitude versus frequency of waveform exposure using a multiple piezoelectric active materials.

FIG. 25 is a schematic and formulaic expression of various dimensional expressions and resultant charge formed in piezoelectric active materials.

FIG. 26 is a schematic flow-chart representing a method for determining respiratory impedance from a subject using a FOT impedance measuring device coupled to a ventilator.

FIG. 27 is a schematic flow-chart varying the position of flow and pressure sensors in the method described in FIG. 26.

FIG. 28 is a schematic flow-chart varying the position of flow and pressure sensors in the method described in FIG. 26.

FIG. 29 is a flow chart of a method to determine baseline Rrs, Xrs, and SDRs, SDXrs and changes in these values in response to a respiratory modulation.

FIG. 30 is a flow chart of a method to determine baseline values of Rrs, Xrs, and SDRs, SDXre and changes in these values in response to a respiratory modulation.

## SUMMARY

The above object is achieved by an actuator with the features of claim 1, by a forced oscillation technique device with the features of claim 10 and by a method with the features of claim 13. One aspect of the disclosure provides an actuator connected to a structural ground of a forced oscillation technique device. The actuator includes an electrical power source, a control device connected to the electrical power source, a first portion including active material connected to the electrical power source, and a second portion including non-active, passive material connected to the first portion. The first portion includes at least one plate-shaped member. The second portion includes a ring member connected to and circumscribing a mesh screen.

In some examples, the active material includes piezoelectric material.

In some implementations, the control device includes one or more of an amplifier and function generator for turning on, turning off or regulating an amount of power provided by the electrical power source for causing oscillating movement of a distal end of the at least one plate-shaped member of the first portion.

In some instances, the second portion further includes an extension member coupler and an extension member. The extension member is connected to the ring member. The extension member coupler is connected to the distal end of the at least one plate-shaped member of the first portion.

In some examples, a proximal end of the at least one plate-shaped member is fixedly-connected to the structural ground of the forced oscillation technique device.

In some implementations, the at least one plate-shaped member includes one plate-shaped member thereby defining the actuator as a single cantilever actuator.

In some instances, the oscillating movement of the distal end of the one plate-shaped member of the first portion causes a corresponding oscillating pivoting motion of the second portion relative the structural ground.

In some examples, the at least one plate-shaped member includes two or more plate-shaped members thereby defining the actuator as a multi cantilever actuator.

In some implementations, the oscillating movement of the distal end of the two or more plate-shaped members of the first portion translates into movement of the extension member coupler along an arcuate path. The movement of the extension member coupler along the arcuate path translates into corresponding oscillating pivoting motion of the extension member, ring member and mesh screen relative the structural ground.

In some instances, the extension member coupler includes an elongated slot defined by opposing first and second end surfaces that extend through a thickness of the extension member coupler.

In some examples, the extension member extends from the structural ground and through the elongated slot of the extension member coupler such that that a distal end of the extension member is arranged beyond a distal end of the extension member coupler.

In some implementations, the extension member is indirectly connected to the two or more plate-shaped members by way of a pin extending entirely through the extension member coupler, the elongated slot and a vertical slot formed by a portion of a length of the extension member that is substantially orthogonal to the elongated slot formed by the extension member coupler.

In some instances, a pair of opposing pins partially extend into a pivoting sleeve member that is pivotally-arranged within the elongated slot of the extension member coupler about a pivot axis that extends through the pair of opposing pins. The extension member is slidably-coupled to the pivoting sleeve member.

In some examples, the electrical power source is connected to a direct current source of power or an alternating current source of power.

Another aspect of the disclosure provides a forced oscillation technique device including a tube-shaped fluid-communicating member, a support member and an actuator. The tube-shaped fluid-communicating member defines a fluid-communicating passage. The support member supports the tube-shaped fluid-communicating member. The support member defines an actuator passage that fluidly intersects the fluid-communicating passage of the tube-shaped fluid-communicating member. The actuator is connected to the support member. The actuator is disposed within the actuator passage and extends into the fluid-communicating passage. The actuator includes: an electrical power source, a control device connected to the electrical power source, a first portion including active material connected to the electrical power source, and a second portion including non-active, passive material connected to the first portion. The first portion includes at least one plate-shaped member. The second portion includes a ring member connected to and circumscribing a mesh screen. The at least one plate-shaped member is movably disposed in the actuator passage. The ring member is movably disposed within the fluid-communicating passage.

In some examples, an upstream opening of the fluid-communicating passage is fluidly in communication with atmospheric pressure.

In some implementations, an upstream opening of the fluid-communicating passage is fluidly in communication with an anesthesia machine or mechanical ventilator.

In some instances, a downstream opening of the fluid-communicating passage is fluidly in communication with a fluid measurement device.

In some examples, the fluid measurement device is a pneumotach.

In some implementations, the pneumotach is communicatively coupled to the control device of the actuator.

In some instances, the fluid measurement device is fluidly in communication with an oral human interface device.

In some instances, the oral human interface device is a breathing tube.

In some instances, the oral human interface device is an endotracheal tube.

In some examples, the fluid measurement device is communicatively coupled to the control device of the actuator.

In yet another aspect of the disclosure provides a method for determining the respiratory impedance (Zrs) of a subject, the method comprising: a. providing a plurality of oscillations generated by a forced oscillation technique impedance measuring device (FIMD) to the airway of the subject, said device comprising : i. an actuator (10, 10', 10") connected to a structural ground (12, 12', 12") of a forced oscillation technique (FOT) impedance measuring device (FIMD) (100, 200, 300, 400, 500, 600, 700, 800, 900, 1000), comprising: ii. an electrical power source (16, 16', 16"); iii. a control device (17, 17', 17") connected to the electrical power source (16, 16', 16"); iv. a first portion (14a, 14a', 14a") including active material connected to the electrical power source, and v. a second portion (14b, 14b', 14b") including non-active, passive material connected to the first portion (14a, 14a', 14a"), wherein the first portion (14a, 14a', 14a") includes vi. at least one plate-shaped member (18, 18', 18"), wherein the second portion (14b, 14b', 14b") includes a ring member (24, 24', 24") connected to and circumscribing a mesh screen (26, 26', 26"); b. obtaining a pressure signal and a flow signal at each of a single, or a plurality of frequencies generated by said mesh screen; c. collecting and processing said pressure signal and flow signal and d. calculating an impedance (Zrs) of the subject from said pressure signal and said flow signal, wherein the frequency ranges from 4Hz to 34 Hz, and the frequency produced by said FIMD is matched to the damped resonance frequency ($\omega d$) of the actuator.

In some implementations, the actuator is a single cantilever actuator

In some examples the actuator is a multi cantilever actuator.

In some instances, the mesh screen produces a peak to peak pressure variation of 0.1kPa. to 0.5 kPa.

## DETAILED DESCRIPTION OF THE INVENTION

The Figures illustrate exemplary embodiments of cantilevered actuators and forced oscillation technique (FOT) devices or otherwise referred to herein as an FOT Impedance Measurement Device (FIMD) and is used synonymously, in accordance with embodiments of the invention. Based on the foregoing, it is to be generally understood that the nomenclature used herein is simply for convenience and the terms used to describe the invention should be given the broadest meaning by one of ordinary skill in the art.

## I. The FOT Impedance Measurement Device (FIMD)

The FIMD as disclosed herein uses an inexpensive lightweight and novel beam bending piezoelectric based actuator for the purposes of measurement of respiratory system impedance, using an oscillation approach known as the FOT. The FIMD has advantages over prior art impedance measurement devices in that it represents a less expensive to manufacture and much simpler mechanical actuator design than other designs. One embodiment of the design takes advantage of the natural resonance of the actuator to maximize oscillation effciency. The actuators of the invention can be used at resonance or near resonance frequency and thus achieve a very high efficiency at very low weight and cost. In some embodiments, the FIMD comprises a multi-layer assemblage to produce increased force during oscillation, which does not need to function at resonance and is designed to work over a range of oscillation frequencies.

The FIMD is used to measure impedance related to difficulty breathing or moving air into and out of the lungs. In one embodiment the device is employed as an aid to the diagnosis of lung disease. In another embodiment the FIMD is used to measure the effectiveness of therapy. In some embodiments, the FIMD is used to monitor respiratory system impedance of sleeping or anaesthetized patients. In another embodiment the FIMD is used to monitor a subject being ventilated or on a ventilator. In another embodiment the FIMD is integrated into a ventilator system. Used with a ventilator the FIMD could help decide the best time to begin weaning from ventilation, based on making assessments of the subject's respiratory system resistance. In some embodiments, the FIMD can be used to make adjustments to the pressure or flow-rate of the ventilator in a proportional assist ventilation to provide aid to the patient in overcoming their respiratory system resistance or reactance. The FIMD could also be used to make adjustments to the pressure or flow-rate of the ventilator to reduce the subject's respiratory system resistance for example to adjust it to approach within 200% of normal respiratory system resistance of healthy controls. Similarly The FIMD could also be used to make adjustments to the pressure or flow-rate of the ventilator to increase the subject's low frequency reactance to within some difference from normal respiratory system of healthy controls such as 0.1 kPa/L/s. Thus, a further embodiment is a method of monitoring a subject on a ventilator to determine the level of impedance at a plurality of time points whereby the data generated is used to adjust the setting of the ventilator. In certain embodiments the adjustment is used to wean the subject from ventilation.

Because of its cost and size advantage, the device can be implemented as a diagnostic device or monitor in health care centers, at home or remotely by a telemedicine system. Its light weight and inexpensive cost make this device a major breakthrough to measurement of lung health in such diseases as asthma and chronic obstructive pulmonary disease (COPD). The technology could be embodied in a diagnostic handheld device or as an attachment to the breathing circuit of an anesthesia machine or a mechanical ventilator.

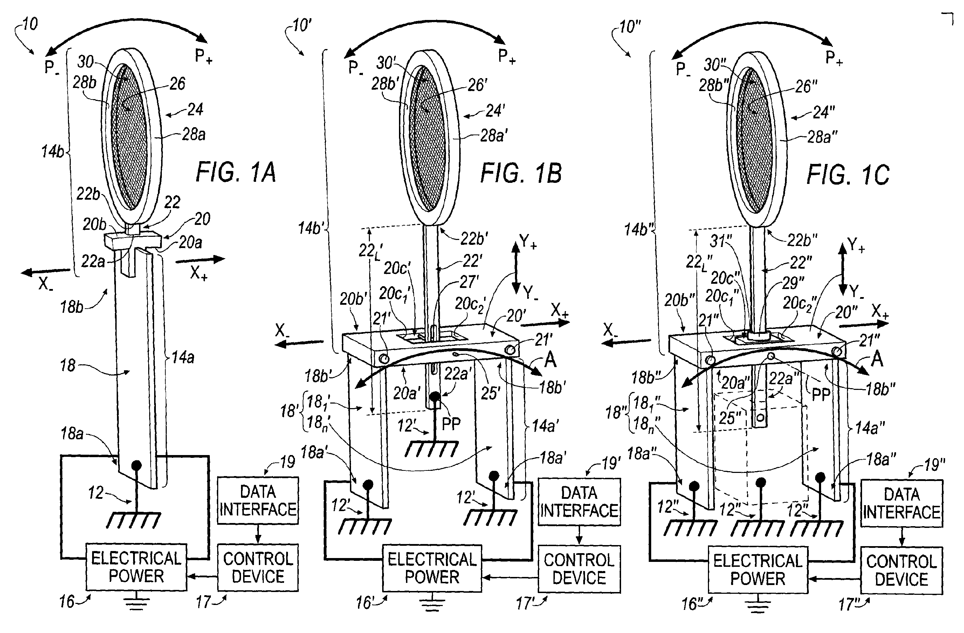

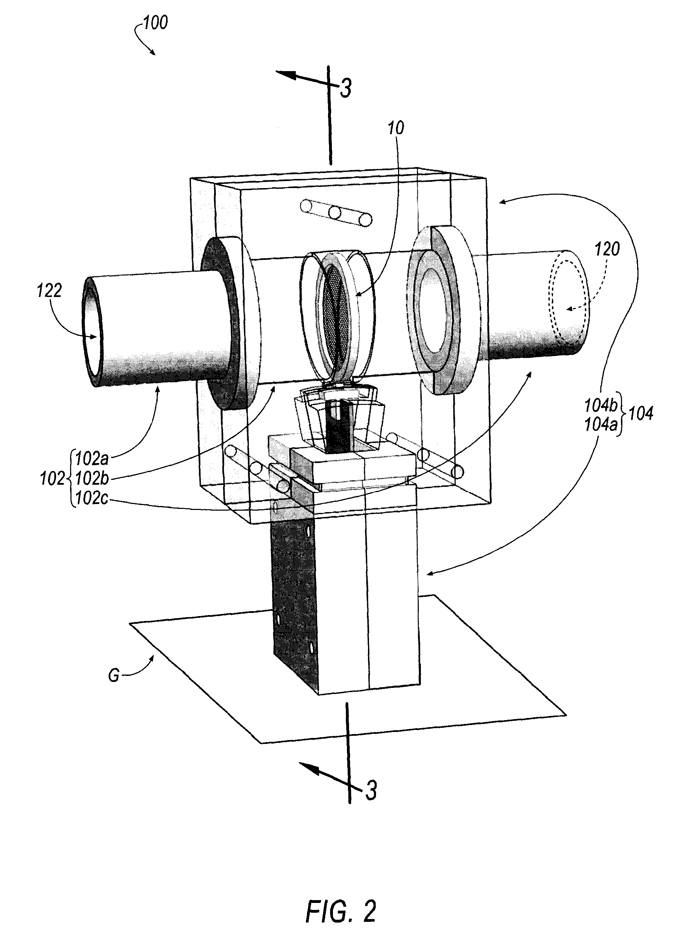

Referring to FIG. 1A, a representative view of an actuator 10 is shown according to an embodiment. The actuator 10 may be a sub-system that is incorporated into a FIMD 100, 200 as seen in, for example, FIGS. 2-5C and 6A-8B, respectively. The FIMD 100, 200 may include a support member (see, e.g., 104, 204, respectively) that acts as a structural ground 12 for the actuator 10.

The actuator 10 may include, for example, a first portion 14a and a second portion 14b. The first portion 14a is connected to the second portion 14b. As will be described in the following disclosure, the first portion 14a includes a single plate-shaped member 18 connected to the structural ground 12, which thereby defines the actuator 10 as a "single cantilever" actuator.

The first portion 14a may be composed of an active material such as, for example, a piezoelectric material. The second portion 14b may be composed of a non-active, passive material.

The first portion 14a may be connected to an electrical power source 16. The electrical power source 16 may include a direct current (DC) source of power or an alternating current (AC) source of power.

The electrical power source 16 may also be connected to a control device 17. The control device 17 is communicatively-coupled to a data interface 19; the data interface 19 may permit an external device (e.g., an external memory device, visual display such as a monitor, a touch screen, an audible annunciator such as a speaker) to be selectively communicatively coupled to the actuator 10 in order to (electronically, visually and/or audibly) obtain readings, measurements and the like from one or more of the actuator 10 and a corresponding FIMD that includes the actuator 10. The control device 17 may permit one of manual control or automatic control over the actuator 10. In some implementations, the control device 17 may permit both of manual control and automatic control over the actuator 10. In order to permit manual control over the actuator 10, the control device 17 may include one or more operator input portions (e.g., buttons, switches, a touch screen or the like) that turn on, turn off or regulate (by way of, for example, one or more of an amplifier and function generator) an amount of power provided by the electrical power source 16 to the first portion 14a. In order to permit automatic control over the actuator 10, the control device 17 may include, for example, software stored in a memory and executable on a processor that turns on, turns off or regulates (by way of, for example, one or more of an amplifier and function generator) an amount of power provide by the electrical power source 16 to the first portion 14a.

Upon activating the electrical power source 16 with the control device 17, the active (e.g., piezoelectric) material composing the first portion 14a may be excited, which thereby translates into oscillating positive movement (see, e.g.,

$X_+$) and negative movement (see, e.g., $X_-$) of the first portion 14a; as a result of activating the electrical power source 16, the movement (e.g., $X_+$ / $X_-$) of the first portion 14a may cause movement (see, e.g., $P_+$ / $P_-$), of the second portion 14b relative to the structure ground 12. The movement, $P_+$ / $P_-$, of the second portion 14a relative the structural ground 12 may be, for example, a positive pivoting motion, $P_+$, and, equally-and-oppositely, a negative pivoting motion, $P_-$, corresponding to the oscillating positive movement (see, e.g., $X_+$) and negative movement (see, e.g., $X_-$) of the first portion 14a.

In an example, the first portion 14a may include a plate-shaped member of active material 18 having a proximal end 18a and a distal end 18b. The proximal end 18a of the plate-shaped member of active material 18 is fixed-connected to (e.g., clamped to) the structural ground 12. The plate-shaped member of active material 18 is connected to the electrical power source 16 as described above. In an embodiment, the active material defining plate-shaped member 18 may be a piezoelectric material.

In an example, the second portion 14b may include an extension member coupler 20 and an extension member 22. The extension member coupler 20 may include a proximal end 20a and a distal end 20b. The extension member 22 may include a proximal end 22a and a distal end 22b. The distal end 18b of the plate-shaped member of active material 18 is directly connected to the proximal end 20a of the extension member coupler 20. The distal end 20b of the extension member coupler 20 is directly connected to the proximal end 22a of the extension member 22. As described above, the second portion 14b is composed of a non-active, passive material; as a result, activation of the electrical power source 16 does not excite the non-active, passive material composing the extension member coupler 20 and the extension member 22.

In an example, the second portion 14b may further include a ring member 24 and a mesh screen 26. The ring member 24 may be defined by a substantially circumferential exterior surface 28a and a substantially circumferential interior surface 28b. The substantially circumferential interior surface 28b may define an opening or passage 30 extending through the ring member 24. The mesh screen 26 may be disposed within the opening or passage 30 and is directly connected to the substantially circumferential interior surface 28b of the ring member 24. The distal end 22b of the extension member 22 is directly connected to the substantially circumferential exterior surface 28a of the ring member 24. As described above, the second portion 14b is composed of a non-active, passive material; as a result, activation of the electrical power source 16 does not excite the non-active, passive material composing the ring member 24 and the mesh screen 26.

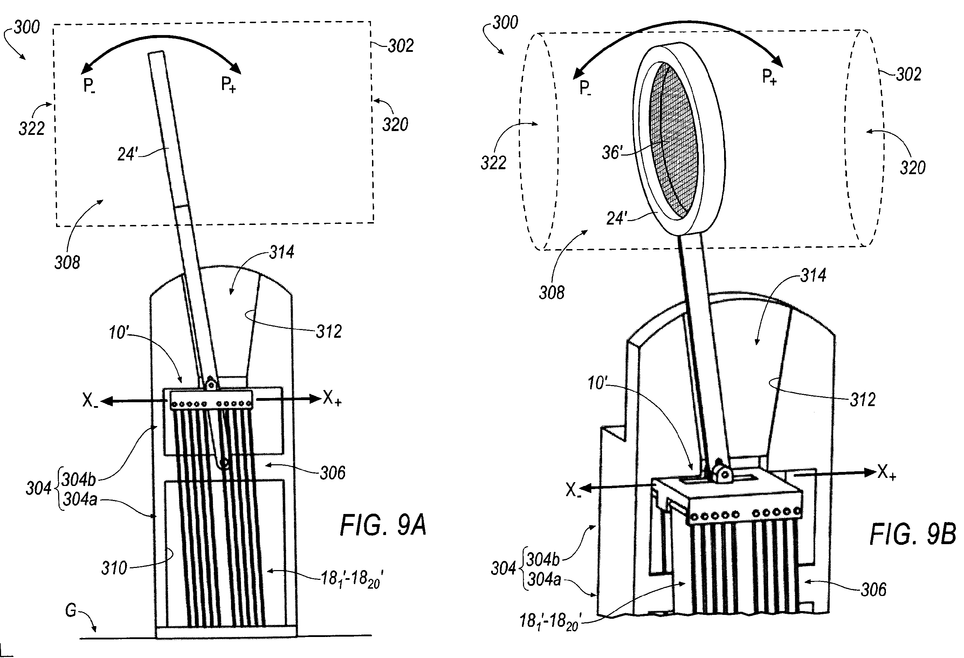

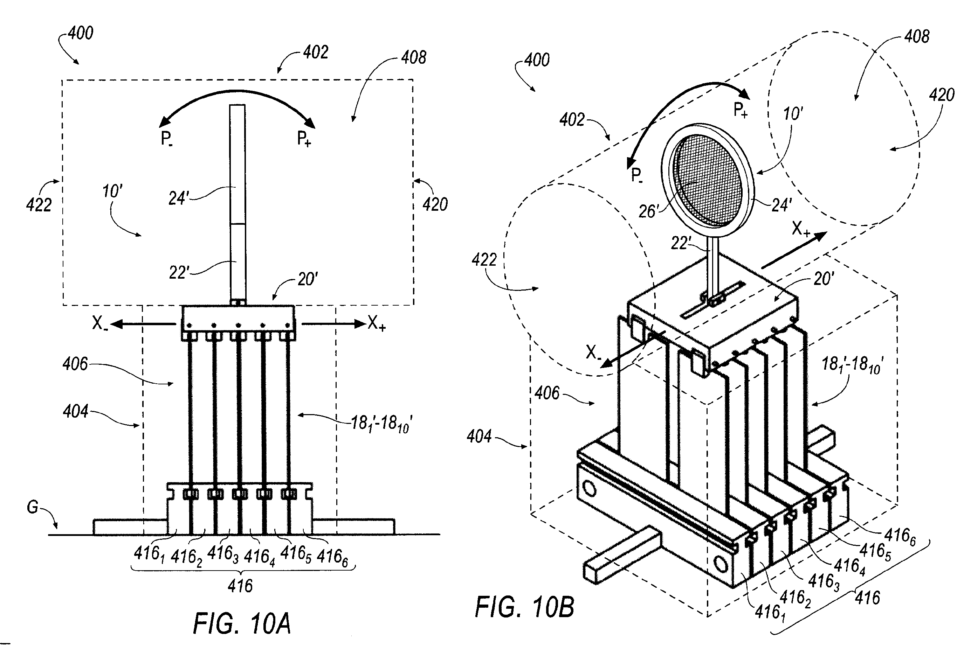

Referring to FIG. 1B, a representative view of an actuator 10' is shown according to an embodiment. The actuator 10' may be a sub-system that is incorporated into any of the FIMDs 300, 400 as seen in, for example, FIGS. 9A-9B and 10A-10B, respectively. Each of the FIMDs 300, 400 may include a support member (see, e.g., 304, 404) that acts as a structural ground 12' for the actuator 10'.

The actuator 10' may include, for example, a first portion 14a' and a second portion 14b'. The first portion 14a' is connected to the second portion 14b'. As will be described in the following disclosure, the first portion 14a' includes a plurality of plate-shaped members 18'; the plurality of plate-shaped members may include a first plate-shaped member $18_1$' and a last (or "$n^{th}$") plate-shaped member $18_n$'. The plurality of plate-shaped members 18' are fixedly-connected to (e.g., clamped to) the structural ground 12', which thereby defines the actuator 10' as a "multi cantilever" actuator.

The first portion 14a' may be composed of an active material such as, for example, a piezoelectric material. The second portion 14b' may be composed of anon-active, passive material.

The first portion 14a' may be connected to an electrical power source 16'. The electrical power source 16' may include a direct current (DC) source of power or an alternating current (AC) source of power.

The electrical power source 16' may also be connected to a control device 17'. The control device 17' is communicatively-coupled to a data interface 19'; the data interface 19' may permit an external device (e.g., an external memory device, visual display such as a monitor, an audible annunciator such as a speaker) to be selectively communicatively coupled to the actuator 10' in order to (electronically, visually and/or audibly) obtain readings, measurements and the like from one or more of the actuator 10' and a corresponding FIMD that includes the actuator 10'. The control device 17' may permit one of manual control or automatic control over the actuator 10'. In some implementations, the control device 17' may permit both of manual control and automatic control over the actuator 10'. In order to permit manual control over the actuator 10', the control device 17' may include one or more operator input portions (e.g., buttons, switches, a touch screen or the like) that turn on, turn off or regulate (by way of, for example, one or more of an amplifier and function generator) an amount of power provide by the electrical power source 16' to the first portion 14a'. In order to permit automatic control over the actuator 10', the control device 17' may include, for example, software stored in a memory and executable on a processor that turns on, turns off or regulates (by way of, for example, one or more of an amplifier and function generator) an amount of power provide by the electrical power source 16' to the first portion 14a'.

Upon activating the electrical power source 16' with the control device 17', the active (e.g., piezoelectric) material composing the first portion 14a' may be excited, which thereby translates into oscillating positive movement (see, e.g., $X_+$) and negative movement (see, e.g., $X_-$) of the first portion 14a'; as a result of activating the electrical power source 16', the movement (e.g., $X_+$ / $X_-$) of the first portion 14a' may cause movement, $P_+$ / $P_-$, of the second portion 14b' relative to the structure ground 12'. The movement, $P_+$/ $P_-$, of the second portion 14a' relative to the structural ground 12' may be, for example, a positive pivoting motion, $P_+$, and, equally-and-oppositely, a negative pivoting motion, $P_-$, corresponding to the oscillating positive movement (see, e.g., $X_+$) and negative movement (see, e.g., $X_-$) of the first portion 14a'.

In an example, the first portion 14a' may include the plurality of plate-shaped members of active material 18' each having a proximal end 18a' and a distal end 18b'. The proximal end 18a' of the each plate-shaped member of the plurality of plate-shaped members of active material 18' is connected to the structural ground 12'. The plurality of plate-shaped members of active material 18' are connected to the electrical power source 16' as described above. In an embodiment, the active material defining the plurality of plate-shaped members 18' maybe a piezoelectric material.

In an example, the second portion 14b' may include an extension member coupler 20'. The extension member coupler 20' may include a proximal end 20a' and a distal end 20b'. An elongated slot 20c' defined by opposing first and second end surfaces $20c_1$', $20c_2$' may extend through a thickness of the extension member coupler 20'; the thickness of the extension member coupler 20' may be bound by the proximal end 20a' of the extension member coupler 20' and the distal end 20b' of the extension member coupler 20'. The distal end 18b' of each of the two or more plate-shaped members of active material 18' are directly connected to the proximal end 20a' of the extension member coupler 20' by way of, for example, transverse pins 21'. As described above, the second portion 14b' is composed of a non-active, passive material; as a result, activation of the electrical power source 16' does not excite the non-active, passive material composing the extension member coupler 20'.

In an example, the second portion 14b' may also include an extension member 22'. The extension member 22' may include a proximal end 22a' and a distal end 22b'. The extension member 22' may be defined by a length $22_L$' extending between the proximal end 22a' of the extension member 22' and the distal end 22b' of the extension member 22'.

The proximal end 22a' of the extension member 22' may be pivotally connected (see, e.g., pivot point, PP) to the structural ground 12', which is opposite to or opposes the proximal end 20a' of the extension member coupler 20'. The extension member 22' extends from the structural ground 12' and through the elongated slot 20c' and beyond the distal end 20b' of the extension member coupler 20' such that that distal end 22b' of the extension member 22' is arranged beyond the distal end 20b' of the extension member coupler 20'. As described above, the second portion 14b' is composed of a non-active, passive material; as a result, activation of the electrical power source 16' does not excite the non-active, passive material composing the extension member 22'.

In an example, the second portion 14b' may further include a ring member 24' and a mesh screen 26'. The ring member 24' may be defined by a substantially circumferential exterior surface 28a' and a substantially circumferential interior surface 28b'. The substantially circumferential interior surface 28b' may define an opening or passage 30' extending through the ring member 24'. The mesh screen 26' may be disposed within the opening or passage 30' and is directly connected to the substantially circumferential interior surface 28b' of the ring member 24'. The distal end 22b' of the extension member 22' is directly connected to the substantially circumferential exterior surface 28a' of the ring member 24'. As described above, the second portion 14b' is composed of a non-active, passive material; as a result, activation of the electrical power source 16' does not excite the non-active, passive material composing the ring member 24' and the mesh screen 26'.

Comparatively, the single cantilever actuator 10 of FIG. 1A is different from the multi cantilever actuator 10' of FIG. 1B in at least two instances. Firstly, the single cantilever actuator 10 includes only one plate-shaped member of active material 18 whereas the multi cantilever actuator 10' includes a plurality (i.e., for example, two or more) plate-shaped members of active material (see, e.g., $18_1$' and $18_n$'). Secondly, the extension member 22 of the single cantilever actuator 10 is directly connected to the plate-shaped member of active material 18 whereas the extension member 22' of the multi cantilever actuator 10' is indirectly connected to the plate-shaped members of active material $18_1$', $18_n$'. In an example, the indirect connection of the extension member 22' to the plate-shaped members of active material $18_1$', $18_n$' is achieved by a pin 25' extending entirely through: (I) the extension member coupler 20', the elongated slot 20c' and a vertical slot 27' formed by a portion of the length $22_L$' of the extension member 22' that is orthogonal to the elongated slot 20c' formed by the extension member coupler 20'. Therefore, when the plurality of plate-shaped members of active material 18' move in an oscillating manner (see, e.g., $X_+$ / $X_-$), the extension member coupler 20' moves along an arcuate path, A, in a first direction according to the arrow, $X_+$, and, oppositely, in a second direction, $X_-$. The movement of the extension member coupler 20' in either of the directions, $X_+$ / $X_-$, along the arcuate path, A, will result in moving the pin 25' along the arcuate path, A; movement of the pin 25' directly causes the positive pivoting motion, $P_+$, and the negative pivoting motion, $P_-$, of the extension member 22' as the pin 25' slides upwardly (see, e.g. arrow $Y_+$) and downwardly (see, e.g. arrow $Y_-$) within the vertical slot 27'.

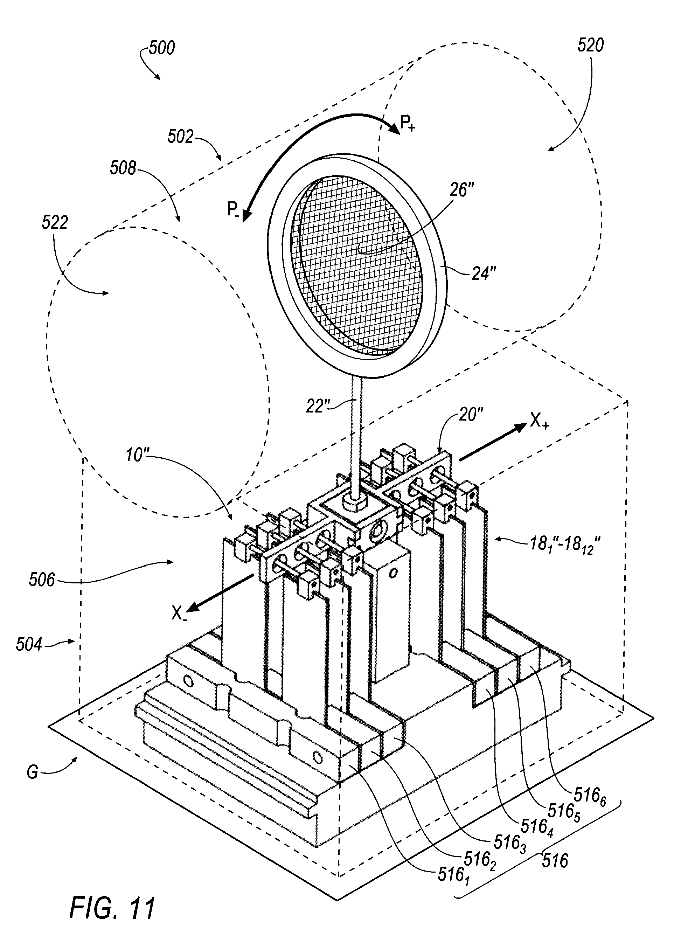

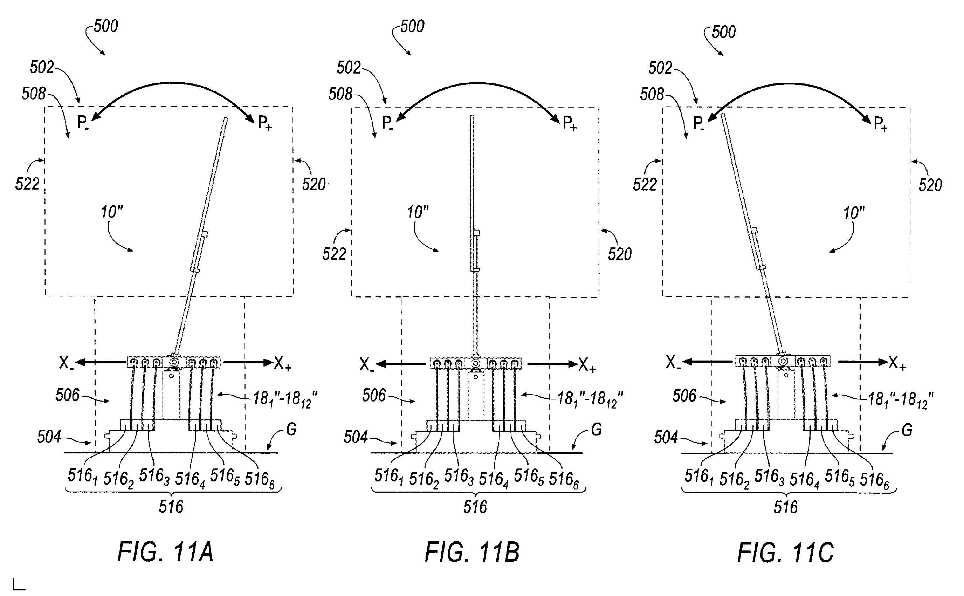

Referring to FIG. 1C, a representative view of an actuator 10" is shown according to an embodiment. The actuator 10" may he a sub-system that is incorporated into any of the FIMDs 500, 600, 700, 800, 900, 1000 as seen in, for example, FIGS. 11, 12, 13, 14, 15 and 16A-16B, respectively. Each of the FIMDs 500, 600, 700, 800, 900, 1000 may include a support member (see, e.g., 504, 604, 704, 804, 904, 1004) that acts as a structural ground 12" for the actuator 10".

The actuator 10" may include, for example, a first portion 14a" and a second portion 14b". The first portion 14a"

is connected to the second portion 14b". As will be described in the following disclosure, the first portion 14a" includes a plurality of plate-shaped members 18"; the plurality of plate-shaped members may include a first plate-shaped member $18_1$" and a last (or "$n^{th}$") plate-shaped member $18_n$". The plurality of plate-shaped members 18" are connected to the structural ground 12", which thereby defines the actuator 10" as a "multi cantilever" actuator.

The first portion 14a" maybe composed of an active material such as, for example, a piezoelectric material. The second portion 14b" may be composed of a non-active, passive material.

The first portion 14a" may be connected to an electrical power source 16". The electrical power source 16" may include a direct current (DC) source of power or an alternating current (AC) source of power.

The electrical power source 16" may also be connected to a control device 17". The control device 17" is communicatively-coupled to a data interface 19"; the data interface 19" may permit an external device (e.g., an external memory device, visual display such as a monitor, an audible annunciator such as a speaker) to be selectively communicatively coupled to the actuator 10" in order to (electronically, visually and/or audibly) obtain readings, measurements and the like from one or more of the actuator 10" and a corresponding FIMD that includes the actuator 10". The control device 17" may permit one of manual control or automatic control over the actuator 10". In some implementations, the control device 17" may permit both of manual control and automatic control over the actuator 10". In order to permit manual control over the actuator 10", the control device 17" may include one or more operator input portions (e.g., buttons, switches, a touch screen or the like) that turn on, turn off or regulate (by way of, for example, one or more of an amplifier and function generator) an amount of power provide by the electrical power source 16" to the first portion 14a". In order to permit automatic control over the actuator 10", the control device 17" may include, for example, software stored in a memory and executable on a processor that turns on, turns off or regulates (by way of, for example, one or more of an amplifier and function generator) an amount of power provide by the electrical power source 16" to the first portion 14a".

Upon activating the electrical power source 16" with the control device 17", the active (e.g., piezoelectric) material composing the first portion 14a" may be excited, which thereby translates into oscillating positive movement (see, e.g., $X_+$) and negative movement (see, e.g., $X_-$) of the first portion 14a"; as a result of activating the electrical power source 16", the movement (e.g., $X_+$ / $X_-$) of the first portion 14a" may cause movement, $P_+$ / $P_-$, of the second portion 14b" relative to the structure ground 12". The movement, $P_+$ / $P_-$, of the second portion 14a" relative to the structural ground 12" may be, for example, a positive pivoting motion, $P_+$, and, equally-and-oppositely, a negative pivoting motion, $P_-$, corresponding to the oscillating positive movement (see, e.g., $X_+$) and negative movement (see, e.g., $X_-$) of the first portion 14a".

In an example, the first portion 14a" may include the plurality of plate-shaped members of active material 18" each having a proximal end 18a" and a distal end 18b". The proximal end 18a" of the each plate-shaped member of the plurality of plate-shaped members of active material 18" is fixedly-connected to (e.g., clamped to) the structural ground 12". The plurality of plate-shaped members of active material 18" are connected to the electrical power source 16" as described above. In an embodiment, the active material defining the plurality of plate-shaped members 18" maybe a piezoelectric material.

In an example, the second portion 14b" may include an extension member coupler 20". The extension member coupler 20" may include a proximal end 20a" and a distal end 20b". An elongated slot 20c" including first and second end surfaces $20c_1$", $20c_2$" may extend through a thickness of the extension member coupler 20"; the thickness of the extension member coupler 20" may be bound by the proximal end 20a" of the extension member coupler 20" and the distal end 20b" of the extension member coupler 20". The distal end 18b" of each of the two or more plate-shaped members of active material 18" are directly connected to the proximal end 20a" of the extension member coupler 20" by pins 21". As described above, the second portion 14b" is composed of a non-active, passive material; as a result, activation of the electrical power source 16" does not excite the non-active, passive material composing the extension member coupler 20".

In an example, the second portion 14b" may also include an extension member 22". The extension member 22" may include a proximal end 22a" and a distal end 22b". The extension member 22" may be defined by a length $22_L$" extending between the proximal end 22a" of the extension member 22" and the distal end 22b" of the extension member 22".

The proximal end 22a" of the extension member 22" may be slidably (according to the direction or arrows, $Y_+$, $Y_-$) connected to the structural ground 12", which is opposite to or opposes the proximal end 20a" of the extension member coupler 20". The extension member 22" extends from the structural ground 12" and through the elongated slot 20c" and beyond the distal end 20b" of the extension member coupler 20" such that that distal end 22b" of the extension member 22" is arranged beyond the distal end 20b" of the extension member coupler 20". As described above, the second portion 14b" is composed of a non-active, passive material; as a result, activation of the electrical power source 16" does not excite the non-active, passive material composing the extension member 22".

In an example, the second portion 14b" may further include a ring member 24" and a mesh screen 26". The ring member 24" may be defined by a substantially circumferential exterior surface 28a" and a substantially circumferential interior surface 28b". The substantially circumferential interior surface 28b" may define an opening or passage 30" extending through the ring member 24". The mesh screen 26" may be disposed within the opening or passage 30" and is directly connected to the substantially circumferential interior surface 28b" of the ring member 24". The distal end 22b" of the extension member 22" is directly connected to the substantially circumferential exterior surface 28a" of the ring member 24". As described above, the second portion 14b" is composed of a non-active, passive material; as a result, activation of the electrical power source 16" does not excite the non-active, passive material composing the ring member 24" and the mesh screen 26".

Comparatively, the multi cantilever actuator 10" of FIG. 1C is substantially similar to the multi cantilever actuator 10' of FIG. 1B but different in at least two instances. Firstly, the extension member 22" of the multi cantilever actuator 10" does not include the vertical slot 27'; rather, the multi cantilever actuator 10" includes a pair of opposing pins 25" that partially extend into a pivoting sleeve member 29" that is pivotally-arranged (about a pivot axis, PP, defined by the pins 25") within the elongated slot 20c" of the extension member coupler 20". Secondly, as a result of the inclusion of the pivoting sleeve member 29", the extension member 22" is permitting to slide upwardly (according to the direction or arrow, $Y_+$) and downwardly (according to the direction or arrow, $Y_-$) within an opening 31" formed by the pivoting sleeve member 29" as the extension member coupled 20" moves (see, e.g., $X_+$, $X_-$) along the arcuate path, A.

Referring to FIG. 2, an FIMD 100 including the single cantilever actuator 10 is shown according to an embodiment. The FIMD 100 includes at least a tube-shaped fluid-communicating member 102 including a downstream segment 102a, an intermediate segment 102b and an upstream segment 102c. One or more of the downstream segment 102a, the intermediate segment 102b and the upstream segment 102c may be supported by a support member 104.

The support member 104 may include a lower body portion 104a and an upper body portion 104b. The lower body portion 104a of the support member 104 may be disposed upon an underlying ground surface, G. The upper body portion 104b of the support member 104 may support and/or contain one or more of the downstream segment 102a, the intermediate segment 102b and the upstream segment 102c.

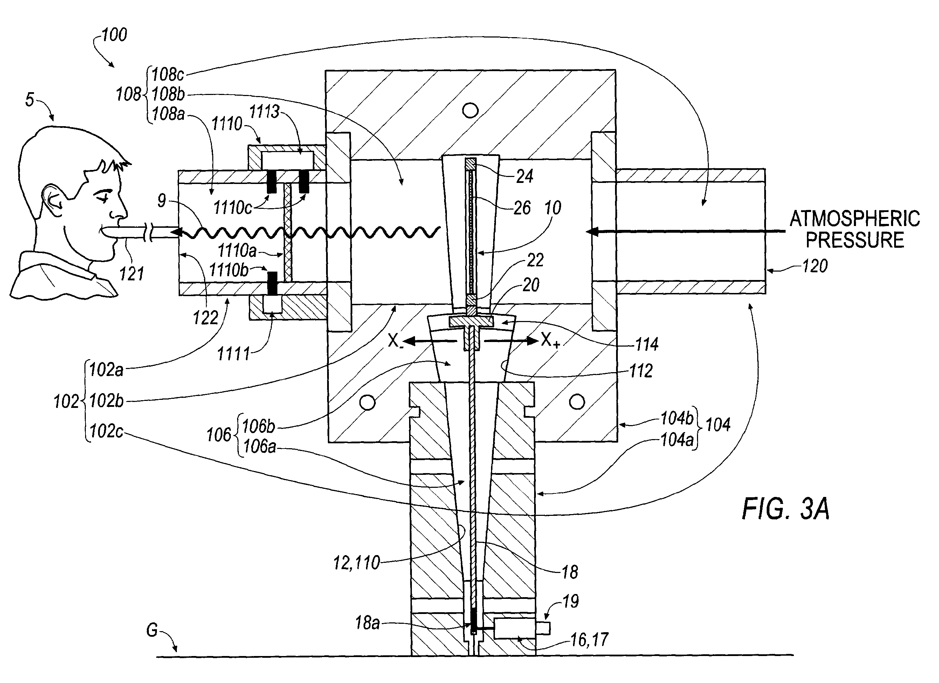

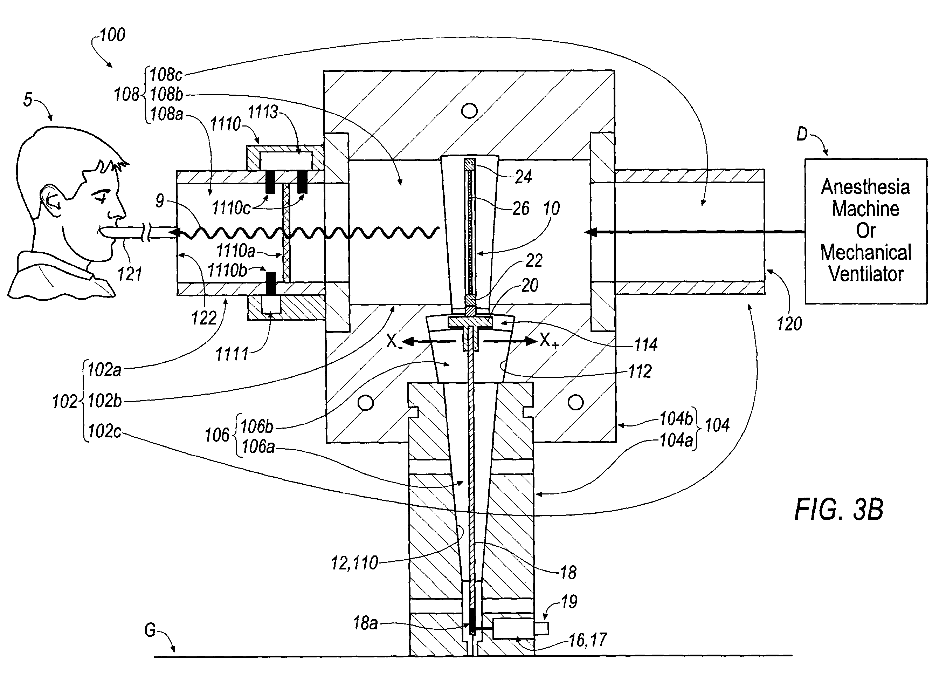

Referring to FIGS. 3A and 3B, the support member 104 may include a passage 106 that is in fluid communication with a passage 108 extending through the tube-shaped fluid-communicating member 102. The passage 108 extending through the tube-shaped fluid-communicating member 102 is substantially orthogonal to the passage 106 extending through the support member 104. The passage 106 formed by the support member 104 may include a first passage segment 106a and a second passage segment 106b. The passage 108 extending through the tube-shaped fluid-communicating member 102 includes a first passage segment 108a, a second passage segment 108b and a third passage segment 108c.

The first passage segment 106a formed by the support member 104 extends through the lower body portion 104a of the support member 104. The second passage segment 106b formed by the support member 104 extends through the upper body portion 104b of the support member 104.

The single cantilever actuator 10 may be disposed within and is connected to the FIMD 100 as follows. In an example, the plate-shaped member of active material 18 of the single cantilever actuator 10 may be movably-arranged within the passage 106 formed by the support member 104. The first passage segment 106a of the passage 106 formed by the support member 104 may be defined by a passage surface 110. The proximal end 18a of the plate-shaped member of active material 18 may be secured to the passage surface 110 that forms first passage segment 106a of the passage 106 formed by the support member 104; thus, the passage surface 110 that forms first passage segment 106a of the passage 106 formed by the support member 104 may be the structural ground 12 for the single cantilever actuator 10 as described above in FIG. 1A.

As seen in FIGS. 3A and 3B, the second passage segment 106b of the passage 106 formed by the support member 104 may be defined by a passage surface 112. The distal end 18b of the plate-shaped member of active material 18 may be movably arranged (according to the direction of arrows $X_+$, $X_-$) within the second passage segment 106b of the passage 106 formed by the support member 104.

The passage surface 112 may further characterize the second passage segment 106b of the passage 106 formed by the support member 104 to include an arcuate channel 114. One or both of the extension member coupler 20 and the extension member 22 of the single cantilever actuator 10 may be movably-arranged within the arcuate channel 114 of the second passage segment 106b of the passage 106 formed by the support member 104.

As seen in FIGS. 3A and 3B, the second passage segment 108b of the passage 108 extending through the tube-shaped fluid-communicating member 102 is generally defined by the intermediate segment 102b of the tube-shaped fluid-communicating member 102. The ring member 24 containing the mesh screen 26 is arranged within the second passage segment 108b of the passage 108 extending through the tube-shaped fluid-communicating member 102.

The second passage segment 108b of the passage 108 extending through the tube-shaped fluid-communicating member 102 may be defined by a passage surface 116. The passage surface 116 may further characterize the second passage segment 108b of the passage 108 extending through the tube-shaped fluid-communicating member 102 to include an arcuate channel 118. The ring member 24 of the single cantilever actuator 10 may be movably-arranged within the arcuate channel 118 of the second passage segment 108b of the passage 108 extending through the tube-shaped fluid-communicating member 102.

As seen in FIGS. 3A and 3B, the upstream segment 102c of the tube-shaped fluid-communicating member 102 may include an upstream opening 120. Referring to FIG. 3A, the upstream opening 120 may be in fluid communication with atmosphere (i.e., atmospheric pressure). Alternatively, referring to FIG. 3B, the upstream opening 120 may be in fluid communication with a device, D, such as, for example, a mechanical ventilator, anesthesia machine or the like.

As seen in FIGS. 3A and 3B, the downstream segment 102a of the tube-shaped fluid-communicating member 102 may include a downstream opening 122. Referring to FIGS. 3A and 3B, the downstream opening 122 may be in fluid communication with pneumotach 1110. Pneumotach 1110 houses a mesh screen 1110a disposed between a pair of flow tubes 1110c. Pneumotach 1110 also comprises a differential pressure sensor 1113. Disposed proximate to pheumotach 1110 is pressure sensor 1111. Either or all of flow tubes 1110c and/or pressure sensor 1111 may also be connected to any one or more of an anti-alias low pass filter (not shown), an analog-to-digital converter (not shown) and an amplifier (not shown). Pneumotach 1110 may be in fluid communication with an oral human interface device, for example a breathing tube 121, or an endotracheal tube 1180 (not shown). A subject, 5, may orally communicate with the breathing tube 121, thereby placing the lungs of the subject, 5, in fluid communication with the passage 108 extending through the tube-shaped fluid-communicating member 102 by way of the breathing tube, 121. The function of the actuator 10 and FIMD 100 with respect to the subject 5, will be described in the methods of use.

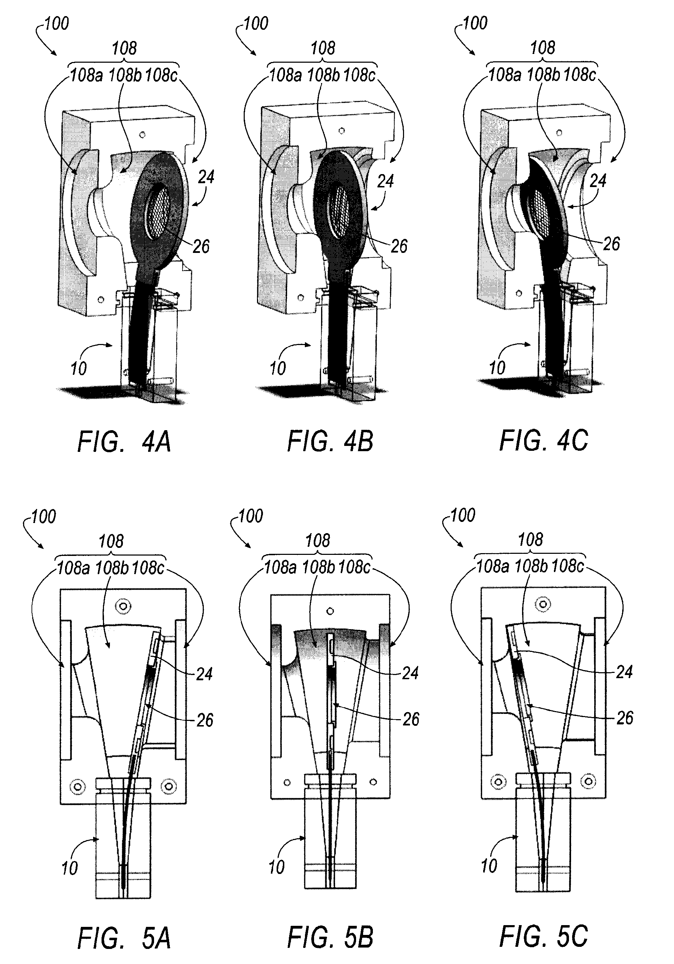

Referring to FIGS. 4A-4C and 5A-5C, a portion of the FIMD 100 is shown; the portion of the FIMD 100 of FIGS. 4A-4C and 5A-5C is generally represented by the single cantilever actuator 10 and the second passage segment 108b of the support member 104. FIGS. 4A and 5A generally show the maximum pivoted orientation of the single cantilever actuator 10 in the positive pivoting motion, P+, with respect to the FIMD 100 such that the ring member 24 is arranged closer to the third passage segment 108c and upstream opening 120 of the FIMD 100. FIGS. 4C and 5C generally show the maximum pivoted orientation of the single cantilever actuator 10 in the negative pivoting motion, P-, with respect to the FIMD 100 such that the ring member 24 is arranged closer to the first passage segment 108a and the downstream opening 122 of the FIMD 100. FIGS. 4B and 5B generally show a neutral pivoted orientation of the single cantilever actuator 10 with respect to the FIMD 100.

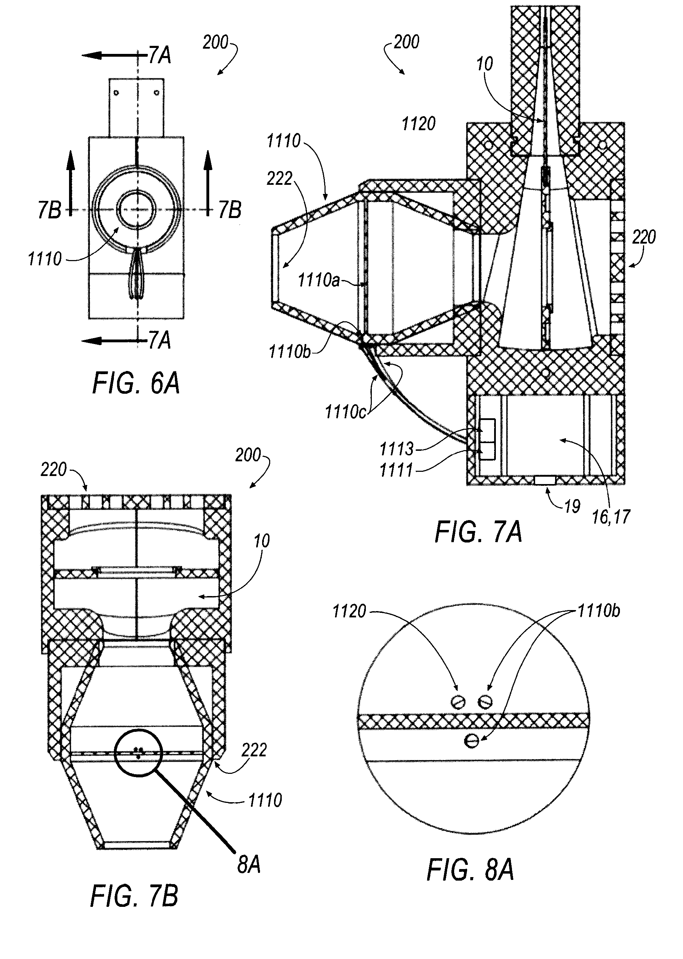

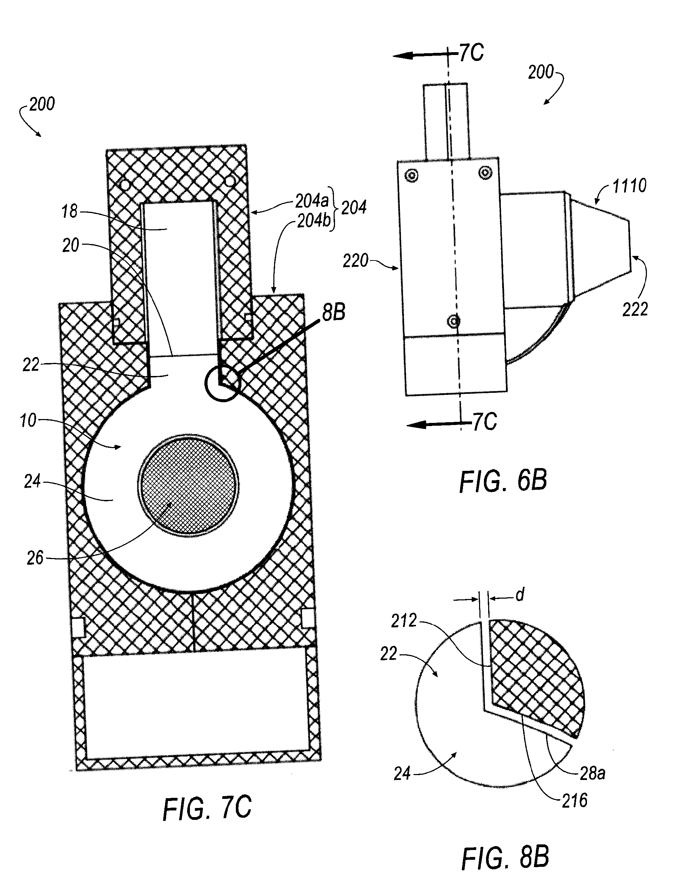

Referring to FIGS. 6A-6B, 7A-7C and 8A-8B, an FIMD 200 including the single cantilever actuator 10 is shown according to an embodiment. Although the FIMD 200 is shown in an inverted orientation with respect to, for example, the FIMD 100, the FIMD 200 may be arranged in a 'right-side-up' orientation that is substantially similar to that as shown in FIGS. 3A-3B with respect to the FIMD 100. The FIMD 200 is substantially similar to the FIMD 100 as described above with the exception that the FIMD 200 is a hand-held device. Similarly, as described above with respect to the FIMD 100, the FIMD 200 includes a fluid-communicating member, F, connected to the downstream opening 222 of the tube-shaped fluid-communicating member 202 of the FIMD 200. The upstream opening 220 of the tube-shaped fluid-communicating member 202 of the FIMD 200 may be in fluid communication with atmospheric pressure.

Referring to FIG. 7A, Pneumotach 1110 houses a mesh screen 1110a and flow ports 1110b. Flow ports 1110b are fluidly connected to flow tubes 1110c. Flow tubes 1110c are in fluid communication with pressure sensor 1111 and differential pressure sensor 1113. Pressure sensor 1111 and/or differential pressure sensor 1113 may also be connected to one or more of an anti-alias low pass filter (not shown), an analog-to-digital converter (not shown) and an amplifier (not shown). Pneumotach 1110 may be in fluid communication with an oral human interface device, for example a breathing tube 121, or an endotracheal tube 1180 (not shown). Referring to FIG. 8A, the pneumotach, 1110, may include a pair of flow ports 1110b and a pressure port 1120. Referring to FIG. 8B, the substantially circumferential exterior surface exterior surface 28a of the ring member 24 and an exterior surface of the extension member 22 may be arranged in a spaced-apart relationship with respect to the passage surfaces 212, 216 of the FIMD at a distance, d; the distance, d, may be approximately equal to 0.50mm.

Referring to FIGS. 9A-9B, an FIMD 300 including the multi cantilever actuator 10' of FIG. 1B is shown according to an embodiment. The FIMD 300 includes a tube-shaped fluid-communicating member 302, which is substantially similar to the tube-shaped fluid-communicating member 102 of FIGS. 2 and 3A-3B; although not explicitly shown at FIGS. 9A-9B, the tube-shaped fluid-communicating member 302 includes a downstream segment, an intermediate segment and an upstream segment that is substantially similar to the downstream segment 102a, the intermediate segment 102b and the upstream segment 102c as shown above at FIGS. 2 and 3A-3B. The tube-shaped fluid-communicating member 302 of the FIMD 300 may be supported by a support member 304.

The support member 304 may include a lower body portion 304a and an upper body portion 304b. The lower body portion 304a of the support member 304 maybe disposed upon an underlying ground surface, G. The upper body portion 304b of the support member 304 may support and/or contain one or more of the downstream segment, the intermediate segment and the upstream segment of the tube-shaped fluid-communicating member 302.

The support member 304 may include a passage 306 that is in fluid communication with a passage 308 that is substantially similar to the passages 106, 108 described above at FIGS. 2 and 3A-3B. The multi cantilever actuator 10' may be disposed within the passages 306, 308 in a substantially similar manner as the single cantilever actuator 10 with respect to the passages 106, 108 of the FIMD 100. For example, the plurality of plate-shaped members of active material 18' of the multi cantilever actuator 10' may be movably-arranged within the passage 306 formed by the support member 304. In an implementation, the plurality of plate-shaped members of active material 18' may include twenty plate-shaped members $18_1'$-$18_{20}'$ arranged in parallel rows of ten plate-shaped members with each row of plate shaped members being separated into two groups of five plate-shaped members.

The passage 306 formed by the support member 304 may be defined by a first passage surface 310. The proximal end 18a' of each plate-shaped member $18_1'$-$18_{20}'$ of the plurality of plate-shaped members of active material 18' may be secured to the passage surface 310; thus, the first passage surface 310 may be the structural ground 12' for the multi cantilever actuator 10' as described above in FIG. 1B.

As seen in FIGS. 9A and 9B, the passage 306 may be further defined by a second passage surface 312. The distal end 18b' of each plate-shaped member $18_1'$-$18_{20}'$ of the plurality of plate-shaped members of active material 18' may be movably arranged (according to the direction of arrows $X_+$, $X_-$) within the passage 306 that is defined by the second passage surface 312. The second passage surface 312 may further characterize the passage 306 formed by the support member 304 to include a channel 314. One or both of the extension member coupler 20' and the extension member 22' of the multi cantilever actuator 10' may be movably-arranged within the channel 314 of the passage 306 formed by the support member 304.

The ring member 24' containing the mesh screen 26' of the multi cantilever actuator 10' is arranged within the second passage segment of the passage 308 extending through the tube-shaped fluid-communicating member 302 in a substantially similar manner as described above with respect to the arrangement of the ring member 24 and mesh screen 26 of the single cantilever actuator 10 with respect to the tube-shaped fluid-communicating member 102 of the FIMD 100. Similarly, the upstream segment of the tube-shaped fluid-communicating member 302 may be in fluid communication with atmosphere (i.e., atmospheric pressure), or, alternatively, with a device, D, such as, for example, a mechanical ventilator, anesthesia machine or the like as described above in a substantially similar manner. Further, the downstream segment of the tube-shaped fluid-communicating member 302 may be in fluid communication with pneumotach 1110. Pneumotach 1110 may be in fluid communication with an oral human interface device, for example a breathing tube 121, or an endotracheal tube 1180 (not shown), in order to permit a subject, 5, to orally communicate with the breathing tube, 121, thereby placing the lungs of the subject, 5, in fluid communication with the passage extending through the tube-shaped fluid-communicating member 302 by way of the breathing tube, 121. The function of the multi cantilever actuator 10' and FIMD 300 with respect to the subject 5, will be described in the methods of use.

Referring to FIGS. 10A-10B, an FIMD 400 including the multi cantilever actuator 10' of FIG. 1B is shown according to an embodiment. The FIMD 400 includes a tube-shaped fluid-communicating member 402 that is substantially similar to the tube-shaped fluid-communicating member 102 as described above at in FIGS. 2 and 3A-3B. The tube-shaped fluid-communicating member 402 may similarly include a downstream segment, an intermediate segment and an upstream segment. One or more of the downstream segment, the intermediate segment and the upstream segment of the tube-shaped fluid-communicating member 402 may be supported by a support member 404 of the FIMD 400.

The support member 404 may be disposed upon an underlying ground surface, G. The support member 404 may support and/or contain one or more of the downstream segment, the intermediate segment and the upstream segment of the tube-shaped fluid-communicating member 402.

The support member 404 may define include a passage 406 that is in fluid communication with a passage 408; the passages 406, 408 are substantially similar to the passages 106, 108 described above at FIGS. 2 and 3A-3B. The passage 408 extending through the tube-shaped fluid-communicating member 402 is substantially orthogonal to the passage 406 extending through the support member 404.

The multi cantilever actuator 10' may be disposed within the passages 406, 408 in a substantially similar manner as the single cantilever actuator 10 with respect to the passages 106, 108 of the FIMD 100. In an example, the plurality of plate-shaped members of active material 18' of the multi cantilever actuator 10' may be movably-arranged within the passage 406 formed by the support member 404. The plurality of plate-shaped members of active material 18' may include ten plate-shaped members $18_1'$-$18_{10}'$ arranged in parallel rows of five plate-shaped members.

A proximal end 18a' of each plate-shaped members $18_1'$-$18_{10}'$ of the plurality of plate-shaped members of active material 18' may be clamped by a pair of adjacent beams $416_1$-$416_6$ that define a plurality of beams 416 of the FIMD 400. The plurality of beams 416 may be defined by six beams $416_1$-$416_6$. Accordingly, the plurality of beams 416 may be the structural ground 12' for the multi cantilever actuator 10' as described above in FIG. 1B.

The distal end 18b' of each plate-shaped member $18_1'$-$18_{10}'$ of the plurality of plate-shaped members of active material 18' may be movably arranged (according to the direction of arrows $X_+$, $X_-$) within the passage 406 formed by the support member 404. One or both of the extension member coupler 20' and the extension member 22' of the multi cantilever actuator 10' may be movably-arranged within a channel formed the passage 406 of the support member 404.

The ring member 24' containing the mesh screen 26' of the multi cantilever actuator 10' is arranged within the passage 408 extending through the tube-shaped fluid-communicating member 402 in a substantially similar manner as described above with respect to the tube-shaped fluid-communicating member 102 at FIGS. 2 and 3A-3B. Similarly, the upstream segment of the tube-shaped fluid-communicating member 402 may be in fluid communication with atmosphere (i.e., atmospheric pressure), or, alternatively, with a device, D, such as, for example, a mechanical ventilator, anesthesia machine or the like as described above in a substantially similar manner. Further, the downstream segment of the tube-shaped fluid-communicating member 402 may be in fluid communication with pneumotach 1110. Pneumotach 1110 houses a mesh screen 1110a and flow ports 1110b. Flow ports 1110b are fluidly connected to flow tubes 1110c. Flow tubes 1110c are in fluid communication with pressure sensor 1111 and differential pressure sensor 1113. Pressure sensor 1111 and/or differential pressure sensor 1113 may also be connected to any one or more of a low pass filter (not shown), an analog-to-digital converter (not shown) and an amplifier (not shown). Pneumotach 1110 may be in fluid communication with an oral human interface device, for example a breathing tube 121, or an endotracheal tube 1180 (not shown), in order to permit a subject, 5, to orally communicate with the breathing tube, 121, thereby placing the lungs of the subject, 5, in fluid communication with the passage extending through the tube-shaped fluid-communicating member 402 by way of the breathing tube, 121. The function of the multi cantilever actuator 10' and FIMD 400 with respect to the subject, 5, will be described in the methods of use.

Referring to FIGS. 11 and 11A-11C, an FIMD 500 including the multi cantilever actuator 10" of FIG. 1C is shown according to an embodiment. The FIMD 500 includes a tube-shaped fluid-communicating member 502 that is substantially similar to the tube-shaped fluid-communicating member 102 as described above at FIGS. 2 and 3A-3B. The tube-shaped fluid-communicating member 502 may include a downstream segment, an intermediate segment and an upstream segment that is substantially similar to the downstream segment 102a, the intermediate segment 102b and the upstream segment 102c of the tube-shaped fluid-communicating member 102. One or more of the downstream segment, the intermediate segment and the upstream segment may be supported by a support member 504 of the FIMD 500.

The support member 504 may be disposed upon an underlying ground surface, G. The support member 404 may support and/or contain one or more of the downstream segment, the intermediate segment and the upstream segment of the tube-shaped fluid-communicating member 502.

The support member 504 may define include a passage 506 that is in fluid communication with a passage 508 in a substantially similar manner as described above with respect to the passages 106, 108 of the tube-shaped fluid-communicating member 102 and the support member 104. The passage 508 extending through the tube-shaped fluid-communicating member 502 is substantially orthogonal to the passage 506 extending through the support member 504.

The multi cantilever actuator 10" may be disposed within the passages 506, 508 in a substantially similar manner as the single cantilever actuator 10 with respect to the passages 106, 108 of the FIMD 100. In an example, the plurality of plate-shaped members of active material 18" of the multi cantilever actuator 10" may be movably-arranged within the passage 506 formed by the support member 504. The plurality of plate-shaped members of active material 18" may include twelve plate-shaped members $18_1$"-$18_{12}$" arranged in parallel rows of six plate-shaped members with each row of plate shaped members being separated into two groups of three plate-shaped members.

A proximal end 18a" of each plate-shaped members $18_1$"-$18_{12}$" of the plurality of plate-shaped members of active material 18" may be clamped by a pair of adjacent beams $516_1$-$516_6$ that define a plurality of beams 516 of the FIMD 500. The plurality of beams 516 maybe defined by six beams $516_1$-$516_6$. Accordingly, the plurality of beams 516 may be the structural ground 12" for the multi cantilever actuator 10".

The distal end 18b" of each plate-shaped member $18_1$"-$18_{12}$" of the plurality of plate-shaped members of active material 18" may be movably arranged (according to the direction of arrows $X_+$, $X_-$) within the passage 506 formed by the support member 504. One or both of the extension member coupler 20" and the extension member 22" of the multi cantilever actuator 10" may be movably-arranged within a channel of the passage 506 formed by the support member 504.

The ring member 24" containing the mesh screen 26" is arranged within the passage 508 extending through the tube-shaped fluid-communicating member 502 in a substantially similar manner as described above with respect to the tube-shaped fluid-communicating member 102 at FIGS. 2 and 3A-3B. Similarly, the upstream segment of the tube-shaped fluid-communicating member 502 may be in fluid communication with atmosphere (i.e., atmospheric pressure), or, alternatively, with a device, D, such as, for example, a mechanical ventilator, anesthesia machine or the like as described above in a substantially similar manner. Further, the downstream segment of the tube-shaped fluid-communicating member 502 may be in fluid communication with an oral human interface device 121, such as, for example, an breathing tube in order to permit a subject, 5, to orally communicate with the breathing tube, 121, thereby placing the lungs of the subject, 5, in fluid communication with the passage extending through the tube-shaped fluid-communicating member 502 by way of the breathing tube, 121. The function of the multi cantilever actuator 10" and FIMD 500 with respect to the subject, 5, will be described in the methods of use.

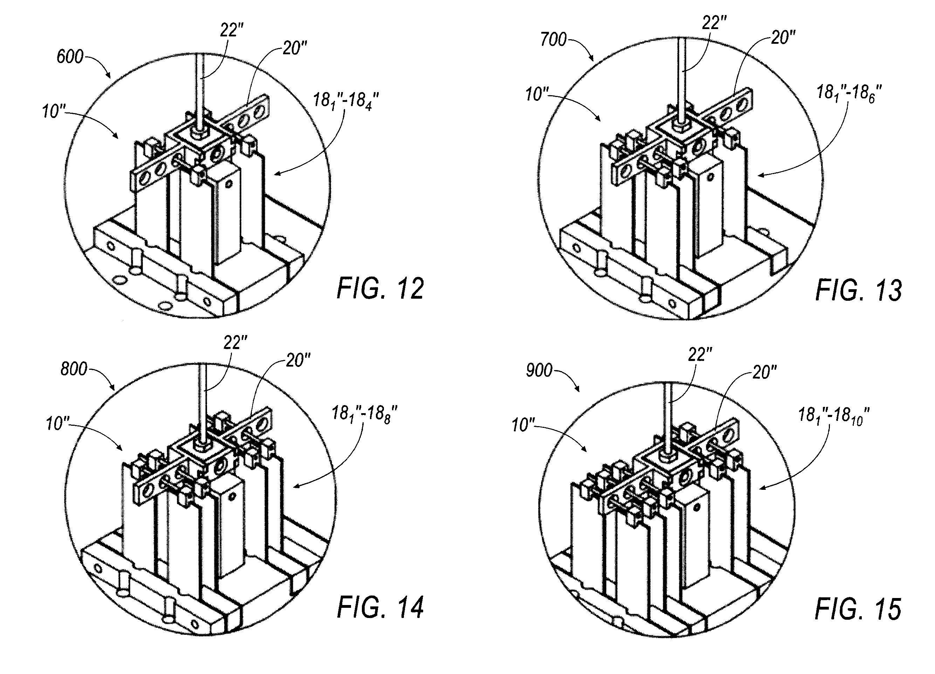

Referring to FIGS. 12-15, FIMDs 600, 700, 800, 900 including the multi cantilever actuator 10" of FIG. 1C are shown according to an embodiment. The FIMDs 600, 700, 800, 900 are partially shown at FIGS. 12-15, but, however, are substantially similar to the FIMD 500 described above at FIGS. 11 and 11A-11C. The FIMDs 600, 700, 800, 900 differ from the FIMD 500 according to the design of the multi cantilever actuator 10".

In an example, the plurality of plate-shaped members of active material 18" of the actuator 10" of the FIMD 600 includes four plate-shaped members $19_1$"-$18_4$" arranged in parallel rows of plate-shaped members. Each row of plate shaped members are separated into two groups of plate-shaped members. Each group of plate-shaped members each includes one plate-shaped member.

In another example, the plurality of plate-shaped members of active material 18" of the actuator 10" of the FIMD 700 includes six plate-shaped members $18_1$"-$18_6$" arranged in parallel rows of plate-shaped members. Each row of plate shaped members are separated into two groups of plate-shaped members. A first group of plate-shaped members includes two plate-shaped members, and, a second group of plate-shaped members includes one plate-shape member.

In an example, the plurality of plate-shaped members of active material 18" of the actuator 10" of the FIMD 800 includes eight plate-shaped members $18_1$"-$18_8$" arranged in parallel rows of plate-shaped members. Each row of plate shaped members are separated into two groups of plate-shaped members. Each group of plate-shaped members each includes two plate-shaped members.

In another example, the plurality of plate-shaped members of active material 18" of the actuator 10" of the FIMD 900 includes ten plate-shaped members $18_1$"-$18_{10}$" arranged in parallel rows of plate-shaped members. Each row of plate shaped members are separated into two groups of plate-shaped members. A first group of plate-shaped members includes three plate-shaped member, and, a second group of plate-shaped members includes two plate-shape members.

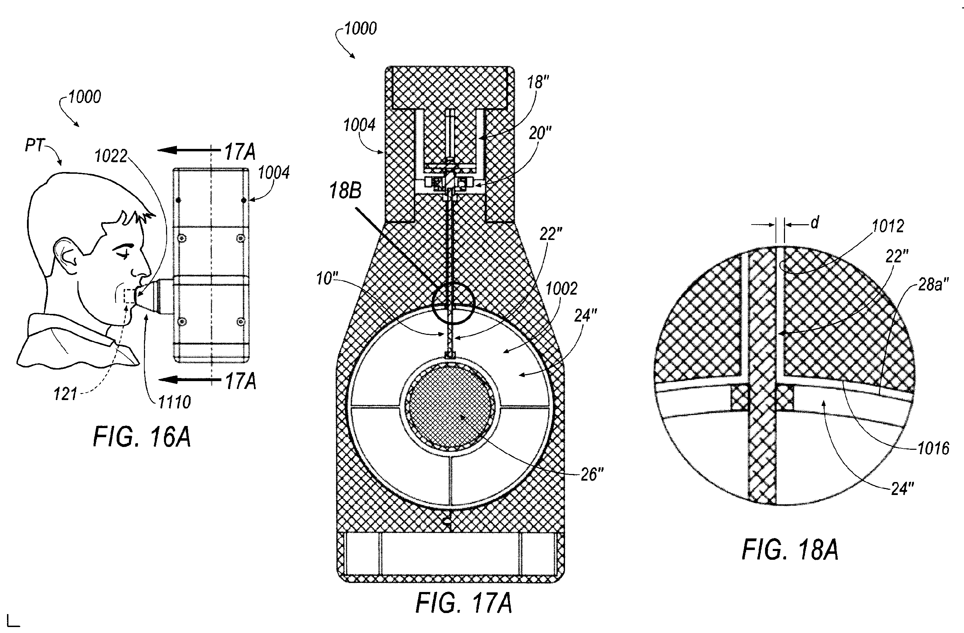

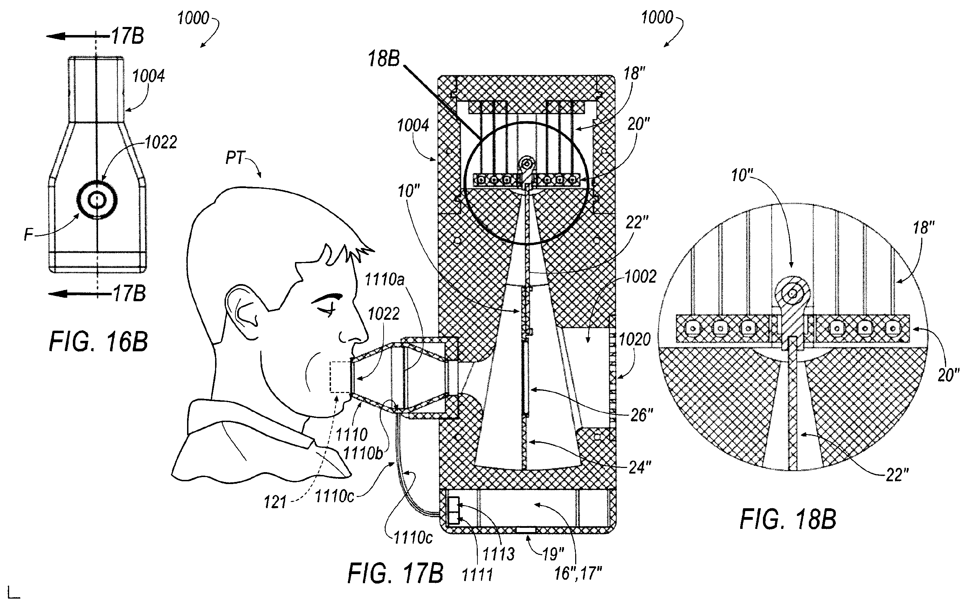

Referring to FIGS. 16A-168, 17A-17B and 18A-18B, an FIMD 1000 including the multi cantilever actuator 10" of FIG. 1C is shown according to an embodiment. Although the FIMD 1000 is shown in an inverted orientation with respect to, for example, the FIMDs 400, 500, 600, 700, 800, 900, the FIMD 1000 may be arranged in a 'right-side-up' orientation that is substantially similar to that as shown in FIGS. 10A-15 with respect to the FIMDs 400, 500, 600, 700, 800, 900. The FIMD 1000 is substantially similar to the FIMD 100 as described above with the exception that the FIMD 1000 is a hand-held device. Similarly, as described above with respect to the FIMD 100, the FIMD 1000 includes a pneumotach, 1110, connected to the downstream opening 1022 of the tube-shaped fluid-communicating member 1002 of the FIMD 1000. Pneumotach 1110 may be in fluid communication with a fluid-communicating member, 121 for example a breathing tube 121, or an endotracheal tube 1180 (not shown). The upstream opening 1020 of the tube-shaped fluid-communicating member 1002 of the FIMD 1000 maybe in fluid communication with atmospheric pressure.

Referring to FIG. 17B, the pneumotach, 1110, may house a mesh screen 1110a. Pneumotach 1110 may include flow ports 1110b which are in fluid communication with a plurality of flow tubes 1110c. Flow tubes 1110c and in fluid communication with pressure sensor 1111, and also differential pressure sensor 1113. Pressure sensor 1111 and/or differential pressure sensor 1113 may also be connected to any one or more of a low pass filter (not shown), an analog-to-digital converter (not shown) and an amplifier (not shown). Referring to FIG. 18A, the substantially circumferential exterior surface exterior surface 28a' of the ring member 24" and an exterior surface of the extension member 22" may be arranged in a spaced-apart relationship with respect to the passage surfaces 1012, 1016 of the FIMD at a distance, d; the distance, d, may be approximately equal to 0.50mm or smaller, to minimize the airgap provided by distance, d. The gap is small so that the resistance to air flow through the gap is much greater than the resistance to airflow through the mesh 36'.

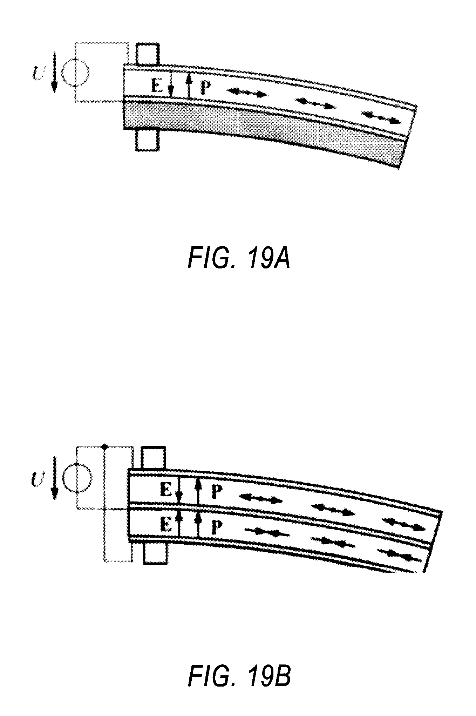

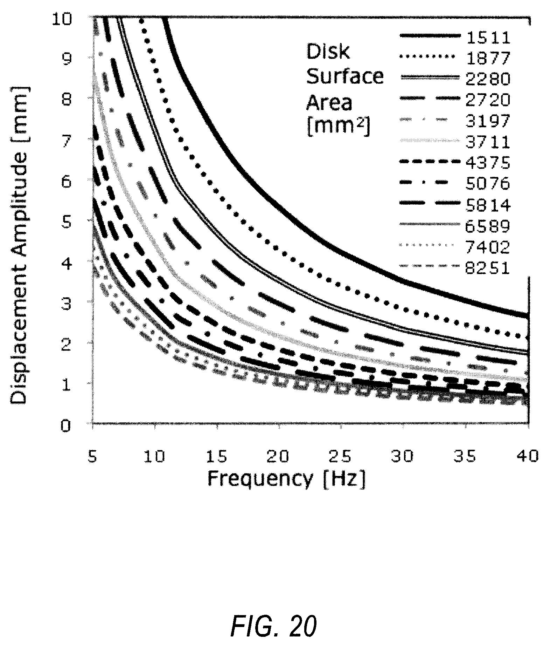

Referring to FIGS 19A and 19B, the piezoelectric active materials of the present invention can include monomorphic or single layer piezoelectric actuators (FIG. 20A), and/or multi-layer piezoelectric actuators commonly referred to as bimorphic piezoelectric actuators (FIG. 20B). Monomorph actuators are made of one active piezoceramic layer and one passive elastic layer. Single layer or multilayer technology on monomorphs refers to the number of piezoelectric layers with electrodes in between. The movement of the piezoceramic active material resulting from its expansion or compression is restricted by the passive elastic component. As a consequence, an internal piezoelectric moment arises deforming the monomorph. The total deflection in z direction is much larger than the deformation of the piezoelectric) active material in x direction even at low voltages (24-200V). In order to increase the deflection of the beam bending actuator, the passive elastic component is replaced by a second active piezoceramic layer to create what is called a bimorph. In this kind of actuator deflections are large, but blocking forces are low, compared to the forces developed by stack actuators. Parallel electrical configuration ensures high sensitivity to input and a bias voltage circuitry prolongs the life of the actuator by eliminating the potential for depolarizing the ceramic layers.

An exemplary electrical connection to a monomorph and bimorph piezoelectric materials are provided in FIGS. 19A and 19B. In some embodiments, useful piezoelectric actuators for use in the device and methods of the present invention can include a standard sized piezoelectric actuator. In an implementation, an exemplary sized piezoelectric biomorph actuator may include a dimension equal to approximately about 6cm x 2cm; however, it will be appreciated that the piezoelectric biomorph actuator is not limited to a particular dimension and that the piezoelectric biomorph actuator may be sized to include any desirable dimension. The actuator secured in a cantilever mounting configuration has a deflection range greater than 2.5 mm, in response to a 150 V (maximum) input, depending of the manufacturer. If deflection is blocked, an actuator will develop a useable force. The performance of these beam bending actuators is affected by the following variables: dimensions, deflection, cantilever mounting: bounded attachment, cantilever mounting: point attachment, parallel drive (high sensitivity to input), bias drive (eliminates potential for depolarization), and mass and geometric characteristics of mechanism attached at the tip.

## II. Method of Use

In some embodiments, a portable FIMD of the present invention produces flow oscillations of varying frequencies at the airway opening during voluntary apnoea. The portable FIMD embodiment can also record the oscillation pressure and flow signals at the subject's respiratory interface or airway opening through the use of a built in pneumotachograph containing pressure and flow sensors/transducers and electronic circuitry, low pass filters, analog-digital converters and microprocessor, fitted with mathematical software to perform various calculations as described herein, including Fourier Fast Transforms of the pressure and flow spectra data to calculate impedance and respiratory resistance and reactance, which can then he used to quantify the impedance of the subject's respiratory system. As used herein, a "subject" can include any mammal having a measurable respiratory function and at least one airway opening that can be used to measure pressure and flow data at the airway opening, including for example, human subjects, non-human subjects such as apes, primates, laboratory animals, such as mice, rats, rabbits, guinea pigs, livestock such as horses, cattle, sheep, pigs, goats, and domesticated animals such as cats, dogs and exotic animals. In some embodiments, the subject is a human subject.

The principle is that the forced oscillations at the airway opening are applied at frequencies greater than the respiration frequency and its harmonics, thus the pressure and flow registered by the pressure and flow sensors either contained within the FIMD or positioned separately thereto, such as pressure and flow sensors within an endotracheal tube, are for the most part, independent of the underlying respiratory pattern. This implies that the driving pressure at the forced oscillation frequency is the pressure attributable to the oscillations in the FIMD since the activity of the muscle pump is negligible at such high frequency. The person's respiratory mechanics at the oscillation frequency can then be determined by the pressure and flow registered at the airway opening even though the recorded pressure and flow signals still contain both the inherent respiratory system pressure and flow and the superimposed forced oscillation signals. Depending on the sites of the P and V' measurements and of the application of the forced oscillations, different kinds of impedance of the respiratory system can be defined. Most commonly, the forced oscillations are applied at the airway opening, and the central airflow (V'ao) is measured with a pneumotach (pneumotachograph) or flow sensors, (for example, pressure and flow transducers) present in the FIMD of the present invention when used as a stand alone device or attached to the mouthpiece, face mask or endotracheal tube when the FIMD is coupled to a ventilator. Pressure can also be sensed at the airway opening (Pao) with reference to body surface (in this case, atmospheric) pressure (Pbs). The impedance of the respiratory system (Zrs) is then the spectral (frequency domain) relationship between transrespiratory pressure (Prs=Pao-Patm) and V'ao: $Zrs(f) = Prs(f)/V'ao(f)$.

In some embodiments, the present invention provides a method for determining the respiratory impedance (Zrs) of a subject. In some embodiments, the method comprises: a providing a plurality of oscillations generated by a forced oscillation technique impedance measuring device (FIMD) to the airway of a subject, wherein the device comprises: i. an actuator (10, 10', 10") connected to a structural ground (12, 12', 12") of a forced oscillation technique (FOT) impedance measuring device (FIMD) (100, 200, 300, 400, 500, 600, 700, 800, 900, 1000), the FIMD comprising: ii. an electrical power source (16, 16', 16"); iii. a control device (17, 17', 17") connected to the electrical power source (16, 16', 16"); iv. a first portion (14a, 14a', 14a") including active material connected to the electrical power source, and v. a second portion (14b, 14b', 14b") including non-active, passive material connected to the first portion (14a, 14a', 14a"), wherein the first portion (14a, 14a', 14a") includes vi. at least one plate-shaped member (18, 18', 18"), wherein the second portion (14b, 14b', 14b") includes a ring member (24, 24', 24") connected to and circumscribing a mesh screen (26, 26', 26") b. obtaining a pressure signal and a flow signal at each of a single, or a plurality of frequencies generated by the mesh screen; c. collecting and processing the pressure signal and flow signal and d. calculating an impedance of the subject from the pressure signal and the flow signal, wherein the frequency ranges from 4Hz to 34 Hz, and the frequency produced by the FIMD is matched to the damped resonance frequency ($\omega$d) of the actuator.

In various embodiments, the FIMD produces small pressure oscillations that is transferred to the subject's airway and then FIMD can record the oscillation pressure and flow signals that can then be used to quantify the impedance, resistance, reactance and variations of these of the subject's respiratory system. Depending on its design, each type of FIMD is capable of generating a characteristic oscillation signal. These external forcing signals maybe mono-frequency, multi-frequency, and may also be applied either continuously (as in the FOT) or in a time-discrete manner (as in the IOS which uses impulses). When the FOT is applied to explore the patterns or mechanisms of frequency dependence of Zrs in health and disease, the simultaneous application of several frequency components, i.e. the use of composite signals, such as pseudorandom noise or recurrent impulses, is preferred. The single-frequency FOT may be used in the tracking of relatively rapid changes in Zrs, e.g. those occurring within the respiratory cycle, or as an accessory device for monitoring airway patency, and it may also be useful in the evaluation of changes in the bronchomotor tone. Embodiments described herein containing a single piezoelectric cantilever arrangement, (See FIG. 1A) can be used for a single frequency measurement using FOT. These devices may constitute low-cost, light-weight, and portable devices that can run off battery power with reliable performance. Exemplary applications of these single piezoelectric cantilever can include: assessment of respiratory mechanics for diagnosis, disease monitory and for determining the effects of bronchoactive agents, for example, in patients and in research laboratories for drug-development using non-human subjects, and/or clinical trials.

In some embodiments, one or more clinical applications of FOT may employ a frequency range that starts from 2-5 Hz, about 10 times higher than the spontaneous breathing rate. More commonly, the lowest frequency is 4, 5 or 6 Hz and can include all frequencies up to approximately 34 Hz or selected frequencies such as 5, 10, 15, 20, 25, 30, 35 Hz, or selected frequencies such as the prime numbers times 2 as 4, 6, 10, 14, 22, 26, 34 Hz. In this frequency range, the healthy respiratory system exhibits a normally frequency independent respiratory resistance (Rrs) whose major component is airway resistance. Respiratory reactance (Xrs) undergoes the transition from negative values to positive values increasing with the frequency. At the characteristic resonant frequency (where Xrs crosses zero) the elastic and inertial forces are equal in magnitude and opposite. Compared to the normal impedance data, in airway obstruction the respiratory resistance measurement in $kPa.s.L^{-1}$ as a function of frequency (0-40 Hz) is higher and negatively frequency-dependent, whereas respiratory reactance measured $kPa.s.L^{-1}$ is lower.

Single-frequency and composite signals (a plurality of frequencies at the same time) are used in clinical practice. But the simultaneous application of several frequencies at the same time is preferred. Therefore, the ability of delivering multiple component signals is a characteristic of the FIMD of the present invention. In several embodiments, the FIMD of the present invention imposes a load against spontaneous breathing of less than 0.1 kPa/L/s below 5 Hz. When using composite signals, for example, as shown in the FIMD of FIG. 10B, the moving mesh 26' should develop a peak-to-peak pressure variation of about 0.1kPa. to about 0.5 kPa, or about 0.2 kPa at the airway opening. In some embodiments, the oscillatory pressure induced from the FIMD or FOT device is preferably not more than 0.5 kPa.

### a. Direct Measurement of Respiratory Impedance

The FIMD of the present invention can be used as a stand alone device to measure various pulmonary function or mechanics, for example, measure and/or monitor lung impedance of sleeping or anesthetized patients, for measuring the effects of bronchoactive agents on predefined lung function for clinical research and/or treatment, and measurement of impedance related to difficulty breathing or moving air into and out of the lungs in a subject with a respiratory disease. In one embodiment, the FIMD is employed as an aid to the diagnosis of a pulmonary disease. In another embodiment, the present FIMD can be used to monitor the respiratory mechanics of a subject with a respiratory disease. In various embodiments, respiratory disease can include, for example, diseases associated with obstructive, restrictive, parenchymal, vascular or infectious respiratory diseases. In some embodiments, such obstructive, restrictive, parenchymal, vascular or infectious respiratory diseases can include one or more of emphysema, bronchitis, asthma, chronic obstructive pulmonary disease (COPD), bronchiectasis, byssinosis, bronchiolitis, asbestosis, fibrosis, cystic fibrosis (CF), sarcoidosis, pleural effusion, hypersensitivity pneumonitis, asbestosis, pleurisy, lung cancer, infant respiratory distress syndrome (IRDS), acute respiratory distress syndrome (ARDS), neurologic diseases affecting the ability of the body to alter respiration rate including spinal cord injury, mechanical diseases affecting pulmonary musculature including myasthenia gravis, and, severe acute respiratory syndrome (SARS), pulmonary edema, pulmonary embolism, pulmonary hypertension, upper respiratory tract infection, including strep throat and the common cold; lower respiratory tract infection, including pneumonia and pulmonary tuberculosis, respiratory neoplasms including mesothelioma, small cell lung cancer, and, non-small cell lung cancer.

In one embodiment, the FIMD may be used to effectively measure a subject's degree of airway (e.g. bronchial) hyperresponsiveness, resistance, reactance and/or impedance and variation of these parameters, for example standard deviation of each of these parameters. In some embodiments, the FIMD of the present invention can also be used to determine the resonant frequency of the subject under analysis, which may be used to assist in understanding the pathologic lung function or mechanics of the subject, and for diagnosing or confirming a particular respiratory disease exemplified herein. In some embodiments, the subject may be a control subject (for example, a healthy subject), a subject performing various lung tests under exercise conditions, or assessment of respiratory impedance in a subject having one or more respiratory diseases as defined herein. In another embodiment, the FIMD may be used to measure a subject being ventilated on a ventilator and as such can be integrated into a ventilation system as shown herein. In various embodiments associated with this aspect, the FIMD of the present invention can be used in a method of monitoring a subject on a ventilator to determine the level of impedance at a plurality of time points whereby the data generated is used determine the level of respiratory system impedance and consequently, to adjust the setting of the ventilator. In certain embodiments, the adjustment is used to wean the subject from ventilation.

The determination of the time course of the respiratory impedance during the spontaneous breathing of a subject can be carried out by applying a pressure signal generated by the FIMD to the airway opening of the subject consisting of the summation of one or more low-amplitude waveforms among which at least one has a frequency ranging from 4-34 Hz. The term "airway opening" is intended to refer to the openings in the mouth, nose, tracheostomy, etc that are exposed to the external environment and through which the subject can inhale and/or exhale.

Prior to actual use in a clinical setting, the FIMD may be calibrated to ensure reliable performance. The calibration should take into account the relative static gain and the relative frequency characteristics of P and V' measuring devices. To check the overall accuracy of the measurement set-up, the use of a reference impedance, whose theoretical impedance is known from physical principles, is recommended. The magnitude of the impedance of the FIMD should be comparable at all measured frequencies to that of the highest Zrs encountered or expected in the measured subject population, i.e. reference impedance with a magnitude of ~1.5 kPa·s·L-1 and ~4 kPa·s·L-1 are suggested for calibration in adult and infant studies, respectively. After proper calibration, a maximum error of about 10% or 0.01 kPa·s·L$^{-1}$, whichever is greater, is allowed over the frequency range of interest.

With reference to FIGs 3A and 3B, in one embodiment, FIMD 100 is used as a stand- alone device in measuring the respiratory impedance in subject 5. In this embodiment, the FIMD 100 can be used to measure the impedance of an airway in a subject 5 under examination. During operation, the FIMD 100 generates and delivers low-amplitude pressure oscillations 9 (approximately ±1-5 cm H$_2$O) at multiple frequencies ranging from 4-34 Hz. at the subject's airway opening using a mouthpiece 121. The signal driving the pressure oscillations can be composed of frequency components for example at 4, 5, 6, 8, 10, 12, 14, 16, 18, 20, 22, 24, 26, 28, 30, 32, and 34 Hz within a one second oscillation period that can be continuously repeated. While different oscillation period durations can be chosen depending on the oscillation frequencies, as long as the oscillation period is an integer multiple of the inverse of all oscillation frequencies, one second can be used in all cases.

In one embodiment of the FOT method using an FIMD, as shown in FIGs. 3A and 3B, measurements on a particular subject 5 can be performed in the sitting position with the head in a neutral or slightly extended position. Flexion of the head should be avoided. During the measurement, the subject 5 (or technician) firmly supports his/her checks and the floor of the mouth using both hands and a nose clip is worn (not shown). The subject 5 is instructed to breathe quietly at FRC level. If the subject 5 is being measured while sleeping or under anesthesia, the subject may be fitted with a respiration mask or an endotracheal tube (not shown). In various embodiments, the subject breathes through a mouthpiece 121 attached to the FIMD 100, or may have an endotracheal tube (not shown) inserted into the trachea of the subject 5. In various embodiments, the subject's airway opening is exposed to mono- or multifrequency oscillatory waveforms 9 applied in a time-discrete manner for two or more recording periods, each period of one to three minutes duration. In some embodiments, the FIMD 100 generates and delivers low-amplitude oscillatory waveforms 9 (approximately ±1-5 cm H$_2$O peak-peak, such that the amplitude of the stimulus signal should not exceed 5 cmH$_2$O to avoid distortions due to the non-linearity of the respiratory system) at one or a multiple of frequencies ranging from 4-34 Hz at the subject's airway opening 6. The oscillatory waveform 9 is generated by the FIMD 100 coupled to a pneumotach 1110 using either a single piezoelectric actuator active material 18 or a multiple piezoelectric actuator active material $18_1$"-$18_{12}$" as shown in FIGs. 3B and 11. Pneumotach 1110 acts a flow sensor to provide flow data during recording. Pressure sensor 1111 measures pressure signals at the airway opening of subject 5 generally positioned at the point of measurement of flow by pneumotach 1110.

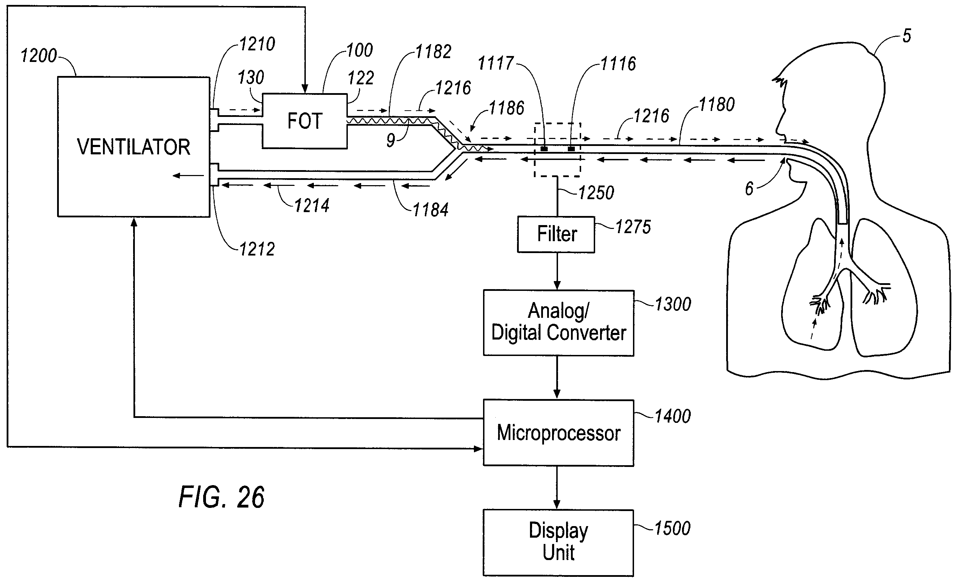

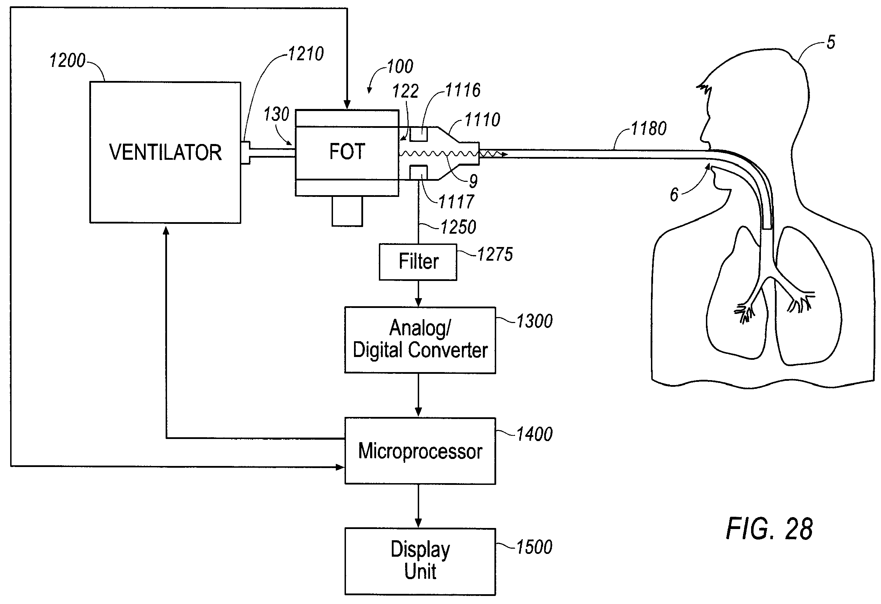

With reference to FIGs. 26-28, the oscillatory waveforms 9 generated by the FIMD 100 and are applied to the airway opening (mouth) 6 of the subject 5 by means of an endotracheal tube 1180 in which one or more flow measuring sensors 1116 (for example, a flow transducer) and one or more pressure measuring sensors 1117 (for example, a pressure transducer) are housed or positioned either downstream of the FIMD 100, or closer to the subject's airway opening 6. In some embodiments, the one or more flow measuring sensors 1116 (for example, a flow transducer, or a mesh screen and a differential pressure sensor) are positioned within the pneumotach 1110 as shown in FIG. 3A, wherein pneumotach 1110 is positioned between the opening to the front end of the FIMD 122 as shown in FIG. 28 and the subject 5 airway opening 6. A pressure sensor 1117 can be positioned proximate to pneumotach 1110 to obtain pressure signals of the subject's airway during recording. Thus pressure and flow are measured, preferably near or at the opening of the subject's airway 6 (for example, mouth, nares, or tracheostomy tube (if a long connection tube is used between the flow sensor 1116 and pressure sensor 1117 and the subject 5 the measurements must be corrected for mechanical properties of the tube). In some embodiments, the flow sensor 1116 and pressure sensor 1117 can be positioned downstream from FIMD 100 in the endotracheal tube 1180 outside the subject 5 airway opening 6, as part of the pneumotach 1110, or at the distal end of the endotracheal tube 1180 near the trachea of the subject 5.

Swallowing, glottis closures, leaking around the mouthpiece, improper seal with the nose clip, irregular breathing or acute hyperventilation during the measurement are reasons to discard the measurement. Most of these events can be detected on the flow signal which should therefore be displayed on the screen during the measurement. If a measurement is considered artcfactual, both Rrs and Xrs should be rejected.

In some embodiments a clinical assessment of a subject' respiratory function and mechanics can involve a total of three to possibly ten technically acceptable measurements using the FIMD of the present invention. The subject should remove the mouthpiece in between successive measurements in order to establish the short-term variability or coefficient of variation (CV) of Zrs in a uniform manner. A further indication of baseline variability may be obtained by repeating the baseline measurements 10-20 min later; which may assist in the interpretation of bronehomotor tests, particularly when Zrs is the sole index used in evaluating bronchial reactivity. Evaluation of a change in Rrs in response to challenge is dependent on the baseline CV value.

In some embodiments, pressure and flow data can be collected using a data acquisition system 1300 (for example, a 150 - 1200 MHz, or a 200, 300, 400, 500, 600, 700, 800, 900, 1000, or a 1200MHz analog to digital data acquisition system) connected to a microprocessor 1400, for example, a computing device containing microprocessor 1400 such as a computer system, operable to store data and perform various calculations required to determine resistance and reactance (and variations thereof, for example standard deviation of resistance and reactance) using Fourier Fast Transformation of the pressure and flow signals produced by the one or more flow measuring sensors 1120 and one or more pressure measuring sensors 1110 over a time domain. For each second of the FOT measurement, Zrs, standard deviation of Zrs, median Rrs, standard deviation of Rrs, median Xrs and standard deviation of Xrs can be calculated and measured at one or more frequencies ranging from 4-34Hz, for example, at single or multiple frequencies, including 4, 5, 6, 8, 10, 12, 14, 16, 18, 20, 22, 24, 26, 28, 30, 32, and 34 Hz. In some embodiments, a bias fan (not shown) can be used to provide approximately 7-15 L/min of fresh air through long stiff walled flexible tube 1190 to permit the subject to breath through a breathing tube 121 having disposed therein a bacterial filter (not shown).

**b. Calculation of Subject's Respiratory Impedance**

In one embodiment, the FIMD can be used to generate oscillatory waveforms at one or more resonance or below resonance frequencies useful in the measurement of a subject's airway compliance. In some embodiments, the FIMD in conjunction with an associated filters and/or analog-digital converter can be used to provide digitized signals to a calculation means, for example a microprocessor, or computer means having appropriate data storage, a central processing unit (cpu), and programmable software to perform various calculations required by the present methods, is operable to measure the resistance (Rrs) and reactance (Xrs) and the variation or standard deviation thereof, by a forced oscillation technique utilizing either a single or a plurality of input frequencies during a plurality of respiratory cycles of a tested subject. In some embodiments, the FIMD in conjunction with a processor or computing apparatus having software instructions to perform various statistical and Fourier transform calculations, can determine the statistical variability (for example, standard deviation) of the Rrs for the subject; and, correlate the statistical variability (for example, standard deviation) of the Rrs of the subject to a standard curve to quantify the degree of airway responsiveness of the subject.

Methods for calculating respiratory impedance using the FOT are well known. For example, Oostveen, E., et al (2003) The Forced Oscillation Technique In Clinical Practice: Methodology, Recommendations And Future Developments, European Respiratory Journal, Vol. 22:1026-1041 provides fundamental theory related to methods for determining a subject's respiratory impedance and methods for calculation of airway reactivity and bronchial hyperresponsiveness using the FOT. In one embodiment, the impedance of the subject's respiratory system (Zrs) is derived from pressure and flow signals obtained from the one or more pressure and flow sensors according to the formulae:

$$Zm = \frac{P(f)}{V(f)} \qquad (1)$$

$$Zrs = \frac{ZcZm}{Zc - Zm} - \frac{ZoZc}{Zo + Zc} \qquad (2)$$

where P($f$) and V($f$) are the Fourier Fast Transforms of pressure and flow respectively of one or more oscillation periods; Zc and Zo are calibrated impedances obtained with the FIMD closed (Zc) and open to the atmosphere (Zo), Zm is a time series of the measured impedance. Equation (1) and (2) are applied for each repeated oscillation period, forming a time series of Zm and Zrs with lengths equal to the number of oscillation periods. If multiple oscillation periods of pressure and flow are used in the Fourier transforms of Equation (1), the length of Zm and Zrs correspondingly decreases by that multiple. Zc and Zo are typically calculated from recordings of up to 1 minute or until coherences of >0.9 or >0.95 are achieved. The correction of Zm by the system impedances compensates for resistive and reactive losses within the FIMD and any filter at the subject attachment as described in "Schuessler TF and Bates JH. A computer-controlled research ventilator for small animals: design and evaluation. IEEE Trans Biomed Eng 42: 860-866, 1995." Other calibration procedures can also be applied that use known calibrated impedances as known loads rather than using Zclosed such as described in Desager KN, Cauberghs M, Van de Woestijne KP. "Two-point calibration procedure of the forced oscillation technique." Med Biol Eng Comput. 1997.

In some embodiments, cycles with inadequate coherence or signal to noise ratio are removed. Rrs and Xrs are the real and imaginary parts of Zrs respectively. Rrs, Rrs and variation (for example standard deviation) in Rrs can be analyzed at different frequencies in subjects and can be performed before and after respiration modulation.

In some embodiments, median Rrs, variation (for example, standard deviation) in Rrs and median Xrs can be calculated from the time series of Zrs and examined over the frequency range 4-34 Hz. The effect of respiratory modulation on mechanical properties of the respiratory system of a subject thus evaluated, can be used to compare the difference in mechanical properties of the respiratory system in normal subjects and subjects with a lung disease, the effectiveness and sensitivity of spirometry, and the correlation of median FOT Rrs, standard deviation of FOT Rrs, and median FOT Xrs on airway hyperresponsiveness.

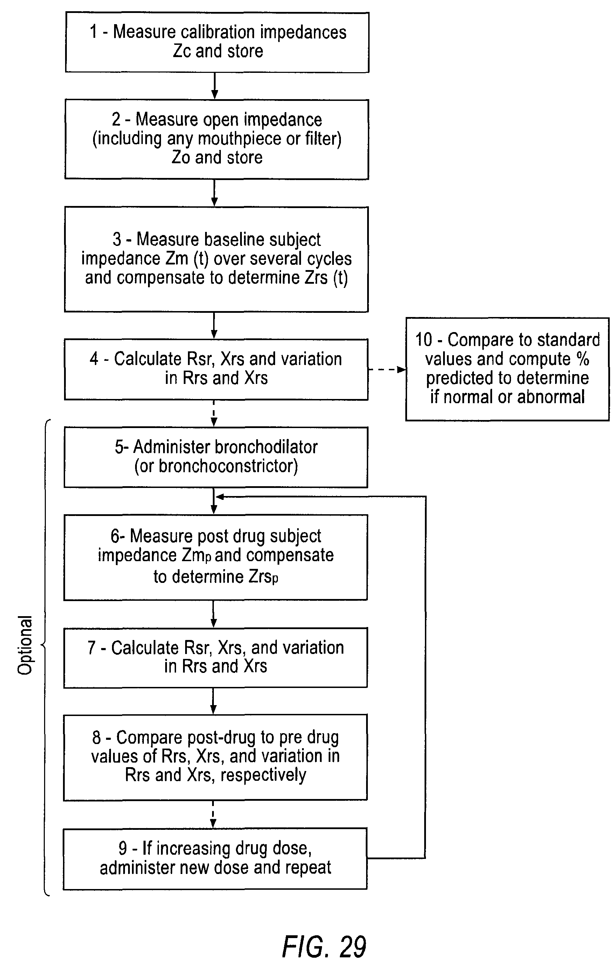

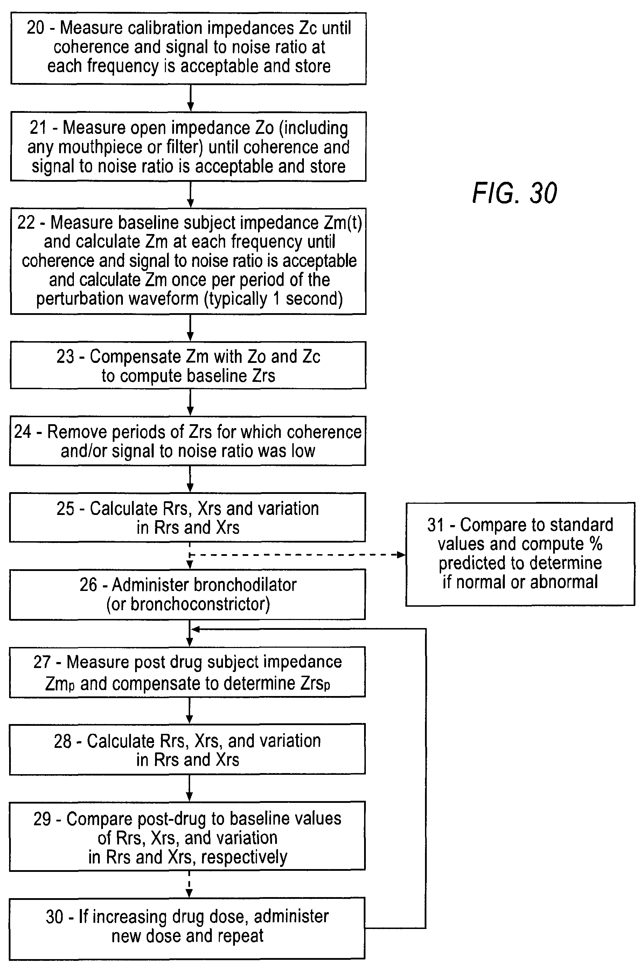

With reference to FIGs. 29-30, a method is described for determining baseline values of Rrs, Xrs and SDRs and the changes in these values over a period of respiratory function assessment, and/or monitoring (for example, 15 minutes, 1 hour, 12 hours, 24 hours, week, 1 month, 1 year, and any time intervals there between). In this embodiment, at step 1, closed impedance Zc (or a calibrated test load impedance) is measured and stored. At step 2, open impedance Zo is measured and stored. At step 3, the baseline subject impedance Zm(t) over several cycles is measured and compensated over several cycles to determine Zrs(t). At step 4, Rrs, Xrs and variations in Rrs and Xrs are measured and if desired are compared (step 10) to standard values to compute % predicted to determine if the Rrs, Xrs and variation in Xrs and Rrs are normal or abnormal. At step 5, a respiration modulation, for example, a bronchoactive agent maybe administered to the subject, or a change in dosed air flow from the ventilator maybe administered. At step 6, the post respiration modulation impedance Zmp is measured and compensated to determine Zrsp. At step 7, Rrs, Xrs and variation in Rrs and Xrs are calculated. At step 8, post- respiration modulation and pre- respiration modulation values of Rrs, Xrs and variations in Rrs and Xrs are measured. Optionally, if the respiration modulation is repeated or altered (for example, a drug dose is increased or repeated) at step 9, steps 6-8 are repeated. In one embodiment, with reference to FIG. 30, an alternate method is described for determining baseline values of Rrs, Xrs and SDRs and the changes in these values in response to a respiration modulation, for example, addition of a bronchoactive agent. In this method at step 20, closed impedance Zc (or a calibrated test load impedance) is measured until coherence and signal to noise ratio at each of a plurality of frequencies is acceptable and stored. At step 21, open impedance Zo is measured until coherence and signal to noise ratio is acceptable (i.e. >0.9, preferably, >0.95) and stored. At step 22, the baseline subject impedance Zm(t) is measured and Zm at each of the plurality of frequencies is calculated once per period of the perturbation waveform. At step 23, Zm is compensated with Zo and Zc to compute baseline Zrs. At step 24, periods of Zrs are removed for which coherence and/or signal to noise ratio was low. At step 25, Rrs, Xrs and variation (for example, standard deviation) in the Rrs and Xrs are calculated and if desired are compared (step 31) to standard values to compute % predicted to determine if normal or abnormal. At step 26, a respiration modulation (for example, a bronchoactive agent may be administered to the subject, or a change in dosed air flow from the ventilator) may be administered. At step 27, post respiration modulation Zmp is measured and compensated to determine Zrsp. At step 28, Rrs, Xrs and variations (for example standard deviation) in Rrs and Xrs are calculated. At step 29, baseline values of Rrs, Xrs and variations (for example, standard deviation) in Rrs and Xrs are compared to post-respiration modulation values. At step 30, if increasing or repeating the respiration modulation, the respiration modulation is administered and steps 27-29 are repeated.

In some embodiments, the generated data on the subject, including any of the respiratory impedance values such as Rrs, Xrs and variation in Xrs and Rrs can be compared to these variables determined from healthy controls not having any pulmonary disease. An overview of the average Rrs values of healthy adult subjects reported from different laboratories is given in table 1. In half of the studies reported by Oostvccn, E., et al (2003), relatively young subjects (an average age of <35 yrs) were investigated; the selection criterion of the subjects was not always reported, or the sample population was limited to a specific subgroup of subjects. Nevertheless, the average Rrs of healthy adults varied little among the different studies, and slightly higher Rrs values were found for females (0.31 kPa·s·L-1) compared with males (0.25 kPa·s·L-1). Prediction equations for the average Rrs and Xrs, and the slope of the Rrs versus f relationship are given in table 2.

Table 1. Overview of the average respiratory resistance Rrs values obtained in healthy adults (Reproduced from Oostveen, E., et al (2003) Table 1. page 1030)

| Cohort | Frequency range (Hz) | Male | | | Female | | |
|---|---|---|---|---|---|---|---|
| | | Rrs kPa.s.L$^{-1}$ | n | Age (yrs) | Rrs kPa.s.L$^{-1}$ | n | Age (yrs) |
| Male Airforce members | 4-24 | 0.25 (0.06) | 224 | 26 (10) | | | |
| Unknown | 8-24 | ~0.26 | 442 | 29 | | | |
| Patients undergoing rehabilitation and healthy hospital workers | 10 | 0.29 (0.08) (M+F) | 102 | 50 | | | |

(continued)

| Cohort | Frequency range (Hz) | Male | | | Female | | |
|---|---|---|---|---|---|---|---|
| | | Rrs kPa.s.L$^{-1}$ | n | Age (yrs) | Rrs kPa.s.L$^{-1}$ | n | Age (yrs) |
| Unknown | 6-24 | 0.26 (0.06) | 126 | 33 (12) | 0.3 (0.06) | 10 0 | 29(12) |
| Subjects referred for lung function testing | 10-32 | 0.26 (0.07) | 32 | 48 (15) | 0.34 (0.07) | 28 | 55 (13) |
| Subjects referred for lung function testing | 6-24 | 0.25 (0.05) | 137 | 53 (14) | 0.31 (0.07) | 14 0 | 58(14) |

Table 2. Prediction equations for the average resistance (Rrs(0)), average reactance (Xrs(0)) and slope of resistance to frequency (Rrs(1)), and the residual SD (RSD) (Reproduced from Oostveen, E., et al (2003) Table 2. page 1030

Male

$$R_{rs}(0)= 0.2454.H+0.001564.W-0.00055.A+0.5919 \text{ Rsd}=0.0493$$

$$R_{rs}(1)= 0.00842.H-0.000047.W-0.000018.A-0.0095 \text{ Rsd}=0.00197$$

$$X_{rs}(0)= 0.1479.H-0.000402.W-0.00022.A-0.1721 \text{ Rsd}=0.0306$$

Female

$$R_{rs}(0)= 0.4300.H+0.00165.W-0.00070.A+0.9312 \text{ Rsd}=0.0619$$

$$R_{rs}(1)= 0.01176.H-0.000106.W-0.000045.A-0.00817 \text{ Rsd}=)0.00256$$

$$X_{rs}(0)= 0.2487.H-0.001700.W-0.00053.A-0.2158 \text{ Rsd}=)0.0406$$

wherein $R_{rs}(0)$ and $X_{rs}(0)$ in kPa•s•L$^{-1}$, $R_{rs}(1)$ in kPa•s$^2$•L$^{-1}$. H:height (m); W: weight (kg); A: age (yrs)

Table 3. Overview of the regression equations of respiratory resistance Rrs values as a function of height obtained in healthy children (Reproduced from Oostveen, E., et al (2003) Table 3. page 1031)

| Frequency band Hz | Subjects n | Age yrs | Rrs kPa•s•L$^{-1}$ | Rsd |
|---|---|---|---|---|
| 15-35 | 16 | 3-5 | $R_{rs15-35}=0.00529xH+1.102$ | |
| 4, 9 | 130 | 3-14 | $R_{rs4}= 2.47-0.013xH$ | |
| 3-10 | 121 | 4-16 | $R_{rs4}= 1.87x10^4 \times H^{-2.12}$ | |
| 2-26 | 138 | 2-16 | $R_{rs6}= 9.2x10^{-5} \times H^2-0.0341xH+3.52$ | 0.15 |
| 2, 4, 12 | 218 | 2-18 | $\log(R_{rs4})- 4.413-2.18 \times \log(H)$ | 10.2% |
| 2-26 | 255 | 2-12 | $R_{rs6}= 0.00017xH^2-0.05407xH=4.77323$ | 0.175 |
| 10 | 377 | 3-18 | $R_{rs10}= 1.392 - 0.00635xH$ | 0.066 |
| 5 | 247 | 3-6.5 | $R_{rs5}= 0.009528xH + 2.0643065$ | |
| 8, 12, 16 | 199 | 3-17 | $\ln(R_{rs8})= 10.990 - 2.370 \times \ln(H)$ | |
| H:height (cm); RSD: residual SD | | | | |

**c. Monitoring Subjects Being Ventilated Or On Ventilators**

In another embodiment the present disclosure provides a method for monitoring the respiratory function of a subject, for example a subject with a respiratory disease using the FIMD (100) of the present invention, when the subject is connected to a ventilator or is being administered an anesthetic. In some embodiments, the method comprises the steps: A method for monitoring the respiratory function of a subject with a respiratory disease assisted with a ventilator, the method comprises the steps: a. ventilating the subject with a respiratory disease with a ventilator set to deliver a volume of fluid at a first flow rate; b. providing a plurality of oscillations generated by a forced oscillation technique impedance measuring device FIMD (100) at the opening of the subject's airway; c. obtaining a pressure signal and a flow signal at each of a single, or a plurality of frequencies generated by said FIMD (100); d. collecting and processing said pressure signal and flow signal; e. measuring the respiratory system resistance (Rrs) of the subject's respiratory system from said pressure signal and said flow signal, wherein the frequency ranges from 4Hz to 34 Hz, and the frequency produced by said FIMD (100) is matched to the damped resonance frequency ($\omega$d) of the actuator; f. comparing said respiratory system resistance from the subject to an average respiratory system resistance of a control population; and g. increasing or decreasing the first flow rate to provide a portion of ventilation assist to overcome a percentage adjustable from 0 to 100% of the subject's respiratory system resistance.

In another embodiment, the present disclosure provides a method for monitoring the respiratory function of a subject, for example a subject with a respiratory disease using the FIMD (100) of the present invention, when the subject is connected to a ventilator or is being administered an anesthetic. In various embodiments, the method comprises the steps: a. ventilating the subject with a respiratory disease with a ventilator set to deliver a volume of fluid at a first flow rate; b. providing a plurality of oscillations generated by a forced oscillation technique impedance measuring device FIMD (100) at the opening of the subject's airway; c. obtaining a pressure signal and a flow signal at each of a single, or a plurality of frequencies generated by said FIMD (100); d. collecting and processing said pressure signal and flow signal; e. measuring the average respiratory system resistance (Rrs) of the subject's respiratory system from said pressure signal and said flow signal, wherein the frequency ranges from 4Hz to 34 Hz, and the frequency produced by said FIMD (100) is matched to the damped resonance frequency ($\omega$d) of the actuator; f. comparing said average respiratory system resistance from the subject to an average respiratory system resistance of a control population; and g. increasing the operating pressure support of the ventilator until said subject's average respiratory system resistance is reduced to within 300%, 200%, 100%, or 50% of said average airway resistance of said control population.

In another embodiment, the present disclosure provides a method for monitoring the respiratory function of a subject, for example a subject with a respiratory disease using the FIMD (100) of the present invention, when the subject is connected to a ventilator or is being administered an anesthetic. In this embodiment, the subject's respiratory impedance is measured and values of reactance are calculated as described herein and compared to a healthy control reactance average. The ventilator is operated in pressure support ventilation mode and can be adjusted accordingly to approach the reactance as determined in a healthy control population. As used herein, the ventilator operating pressure support (also known as pressure support ventilation (PSV)) is a pressure assist form of mechanical ventilatory support that augments the patient's spontaneous inspiratory efforts with a clinician selected level of positive airway pressure. The operating pressure support level is a quantifiable level of delivery of airway pressure commonly known as positive end expiratory pressure (PEEP) having levels that can be quantified in kPa. The patient triggers the ventilator- the ventilator delivers a flow up to a preset pressure limit (for example 10 cmH$_2$O) depending on the desired minute volume, the patient continues the breathe for as long as they wish, and flow cycles off when a certain percentage of peak inspiratory flow (usually 25%) has been reached. Tidal volumes may vary, just as they do in normal breathing.

In some embodiments, the exemplary method comprises the steps: a. ventilating the subject with a respiratory disease with the ventilator set to deliver a volume of a fluid at a first flow rate and a first operating pressure support level; b. providing a plurality of oscillations generated by a forced oscillation technique impedance measuring device FIMD (100) at the opening of the subject's airway; c. obtaining a pressure signal and a flow signal at each of a single, or a plurality of frequencies generated by said FIMD (100); d. collecting and processing said pressure signal and flow signal; e. measuring the average respiratory system low frequency reactance (Xrs) of the subject's respiratory system from said pressure signal and said flow signal, wherein the frequency ranges from 4Hz to 34 Hz, and the frequency produced by said FIMD (100) is matched to a damped resonance frequency ($\omega$d) of the actuator; f. comparing said average respiratory system low frequency reactance from the subject to an average respiratory system low frequency reactance of a control population; and g. increasing the first operating pressure support level of the ventilator until said subject's average respiratory system low frequency reactance is increased to within 0.05 kPa/L/s, 0.1 kPa/L/s, 0.2, kPa/L/s, 0.3 kPa/L/s, or 0.5 kPa/L/s of said average respiratory system low frequency reactance of said control population.

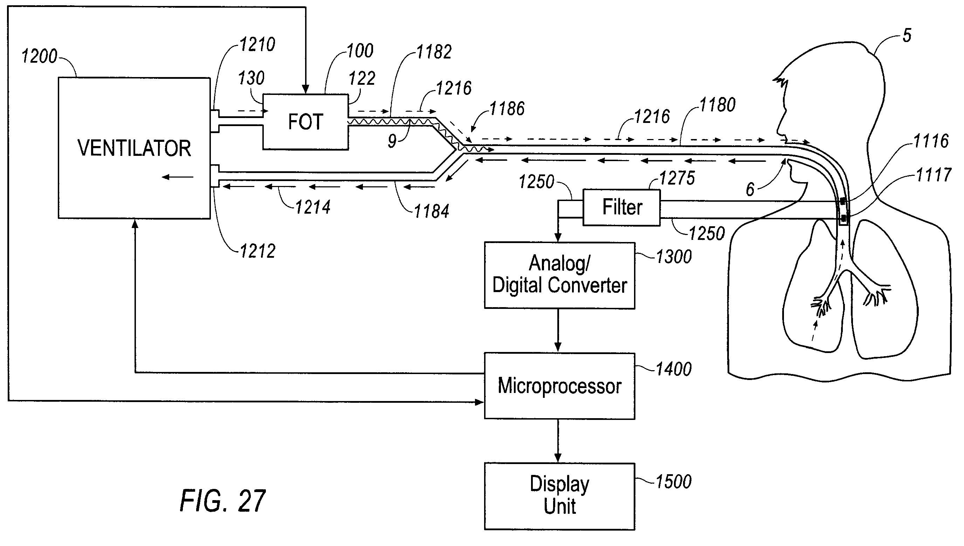

As shown with reference to FIGs. 26-28, a ventilator/monitoring system of the present invention is exemplified, in which a subject 5 is connected to a ventilator 1200 and having an FIMD 100 of the present invention disposed therebetween. In this embodiment, ventilator 1200 can be connected to a FIMD 100 to provide ventilation and monitoring functions for a subject in need thereof. The ventilator can be operated in assist control mode or pressure support ventilation mode. Subject 5 is shown intubated with an endotracheal tube 1180. Endotracheal tube 1180 can be any conventional endotracheal tubes commonly used in the art to deliver air, gasses other than air, or mixtures thereof, including for example, helium, nitric oxide, nitrous oxide, xenon, or certain volatile anesthetic agents such as desflurane, isoflurane, or sevoflurane via positive pressure ventilators. In an exemplary embodiment, endotracheal tube 1280 can have a length and size chosen based on the distance between the FIMD 100 and the subject 5, and the subject's body size, with the smaller sizes being used for pediatric and neonatal subjects. In some embodiments, the internal diameter of the endotracheal tube 1180 can range from about 0.2 m to about 3 m in length and have an internal diameter ranging from about 0.2 cm to about 3.0 cm. In some examples, endotracheal tube 1180 can be constructed of polyvinyl chloride, silicone rubber, latex rubber, or stainless steel. In some embodiments, endotracheal tube 1180 can have an inflatable cuff to seal the trachea and bronchial tree against air leakage and aspiration of gastric contents, blood, secretions, and other fluids. In some embodiments, uncuffed tubes can be used for pediatric patients (in small children, the cricoid cartilage, the narrowest portion of the pediatric airway, often provides an adequate seal for mechanical ventilation). In various embodiments, endotracheal tube 1180 can include oral or nasal, cuffed or uncuffed, preformed (e.g. RAE (Ring, Adair, and Elwyn) tube), reinforced tubes, and double-lumen endobronchial tubes. Commercial endotracheal tubes are available, for example, Microcuff™ from Kimberly Clark Corp. Rosewell, GA USA. In one embodiment, a space within the lumen of endotracheal tube 1180 is the site where the impedance of the subject's respiratory airway is determined and calculated using pressure sensor 1117 and flow sensor 1110. The subject's airway pressure and airway flow using flow sensor 1116 and pressure sensor 1120 is measured in or near the subject 5 airway opening 6. Generally, the flow sensor 1116 and pressure sensor 1117 are positioned proximately, such that the distance between the two sensors do not exceed 5 cm. While other positions can be utilized including pressure sensors 1117 and flow sensors 1116 externally positioned at the distal end of the endotracheal tube as shown in FIG. 27, the pressure sensor 1117 and flow sensor 1116 can be located as close as possible near each other. The endotracheal tube 1180 can be connected via a standard connection to ventilator tubing, arms 1182 and 1184 via a standard valve or connector at 1186. The first arm may be called the expiratory arm 1184, and the second arm may be called the inspiratory arm 1182. Each arm has a proximal end which is generally near the airway opening 6 or subject, the distal end of each arm generally enter the ventilator 1200. In one embodiment, the FIMD 100 is positioned along the inspiratory arm 1182. The FIMD 100 and has a front end 122 and a rear end 130. The direction of flow of air or gas produced by the ventilator 100 passes through the FIMD 100 and travels towards the distal end of the of the inspiratory arm 1182 and then into the subject 5. The subject's expiration travels through the endotracheal tube 1180 and then into the distal end of the expiratory arm 1184 and then into the ventilator 1200.

In one embodiment as shown in FIG. 26, flow sensor 1116 and pressure sensor 1117 are positioned in the endotracheal tube 1180 between the subject 5 and the bifurcation point 1186. The flow sensor 1116 measures the flow of tidal breathing of the subject including the FOT oscillations, and the pressure sensor 1117 measures the pressure of fluid at the measurement point. The signals produced by the flow sensor 1116 and pressure sensor 1117 are sent via an electronic circuit 1250 to a filter 1275, for example an anti-aliasing low-pass filter, and then via circuit 1250 to a data acquisition system 1300 comprising an analog/digital converter, which then digitizes each signal and sends these signals to a microprocessor 1400, which can include a microprocessor or cpu as used in a general purpose computer. The processor may have one or more data buffers with appropriate software programs operable to receive and process the digital signals received from data acquisition system 1300. The microprocessor 1400 can also perform calculations such as Fourier Transformation of the received flow and pressure signals from flow sensor 1116 and pressure sensor 1117. The microprocessor 1400 may also have suitable software to perform various calculations as described herein to determine various parameters of the subject's respiratory impedance, including one or more of Zrs, median Rrs, standard deviation of Rrs, median Xrs, standard deviation of Xrs and resonant frequency of the subject 5 measured at the oscillation frequencies produced by the FIMD.

In some embodiments, the lung impedance thus calculated can be displayed on display unit 1500. Once the subject's 5 airway impedance is displayed or otherwise communicated, the ventilator 1200 can be manipulated by a respiratory therapist, physician or caretaker to increase or decrease the flow of air or gas emanating from the ventilator from valve 1210. In some embodiments, such adjustment of the ventilator 1200 can be controlled manually by an operator, or autonomously using microprocessor 1400 which may be in electrical and/or data communication with ventilator 1200. In some embodiments, when the subject's Zrs, median Rrs, standard deviation of Rrs, median Xrs or standard deviation of Xrs are calculated, a comparing step can be performed by microprocessor 1400 to determine whether the subject's airway impedance is approaching or deviating from stored sex and/or aged matched values of Zrs, median Rrs, standard deviation of Rrs, median Xrs or standard deviation of Xrs calculated from healthy controls (See Tables 1-3). In some embodiments, iterative interrogation of the subject's airway impedance can be used to monitor the function of the subject's airway hyperresponsiveness and/or lung function. The status of the subject's airway hyperresponsiveness and/or lung function can be modulated by varying the amount of air or gas being administered. As the subject's airway impedance approaches normal levels, the subject is weaned from the administered ventilated air or gas, and the flow can be adjusted until the values of one or more of Zrs, median Rrs, standard deviation of Rrs, median Xrs and standard deviation of Xrs approach those calculated from healthy controls. In some embodiments, the ventilator 1200 may also be coupled to other vital sign monitors in addition to the respiratory impedance FIMD 100 to measure and assess cardiac function, blood pressure, brain signals, for example, an ECG device, EEG device, blood pressure device and the like.

## EXAMPLES

### Example 1. Single Piezoelectric Actuator FIMD

The general criteria from Oostveen et al. for FOT clinical practice provide recommended operating parameters for use of the FIMD of the present invention. Design considerations for construction of the FIMD exemplified herein, fluid dynamics, vibration engineering theory and piezoelectric multilayered beam bending actuator practical concepts were used as described in the flowing sections.

The working prototypes of the device were modeled in SOLIDWORKS®. For its construction, the custom parts were machined using computer numerical control (CNC) machining and off-the-shelf components where used to keep the cost of the prototype low.

Design

In one example, the FIMD embodies a moving mesh to impose pressure oscillations of 6 Hz and 19 Hz on top of the breathing of the patients breathing (see Figures 6A-8A). Equation 5 and 6 were used to calculate the displacement (amplitude) at which the mesh disk with area A must move to achieve the chosen pressure and resistance magnitudes at any particular frequency.

$$\delta = P/(R \cdot \omega \cdot A) \qquad (5)$$

$$A = (\pi \cdot r_o{}^2) - (\pi \cdot r_i{}^2 \cdot \gamma) \qquad (6)$$

Where $\delta$ is amplitude at the center of the mesh screen, P is pressure, R is resistance to air flow, $r_o$ is the outer radius of the mesh, $r_i$ is the inner radius and $\gamma$ is the percentage of open area of the wire cloth (mesh). Figure 20 shows the required amplitude of oscillation for different mesh-disk surface areas for an oscillating pressure amplitude of 0.5 $cmH_2O$ and mesh resistance of 0.5 $cmH_2O/L/s$.

To deliver the motion of the mesh disk (See for example, Figure 11A-11C), the configuration of the piezoelectric actuator was chosen to be a cantilever bender bimorph type due to the high displacement capability. A commercially available bimorph piezoelectric actuator, (Cat. No. 40-2010, type: 600/200/0.7SA, APC International Ltd, Mackeyville, PA USA) with the longest available active length (60nm length, 20mm width and 0.70mm thickness) which could achieve a maximum deflection, without mass at the tip, of 2.6 mm at 150 DC Volts and a blocking force of 0.5 N was used. The actuator has a parallel electrical configuration that ensures high sensitivity to input drive and helps prolong the life of the actuator by eliminating the potential for depolarizing the ceramic layers as it uses a bias voltage circuitry.

In order to get larger displacements, the actuator has to be driven at resonance frequency. Since the oscillation frequency is a key aspect of the OS, the system was tuned so the desired frequency for FOT matched the damped resonance frequency ($\omega$d) of the actuator including the mesh-disk affixed on the end of the actuator tip. After some testing of the performance of the actuator by static and quasi-static tests, it was evident that the system was underdamped and the damping ratio was found by the log decrement method and half power method to be $\xi = 0.07$. It was also found that the stiffness k became nonlinear after applying loads greater than 0.3 Newton. Equation 7 was used to calculate a theoretical estimate of $\omega$d as follows,

$$\omega_d = \omega_n \sqrt{1 - \zeta^2} \qquad (7)$$

$$\omega_n = \sqrt{k/m} \qquad (8)$$

where $\omega$n is the natural resonance frequency, k is stiffness, m is mass and is the experimentally determined damping ratio as described in Inman, D. Engineering vibration. Upper Saddle River, N.J. Prentice Hall. 2001.

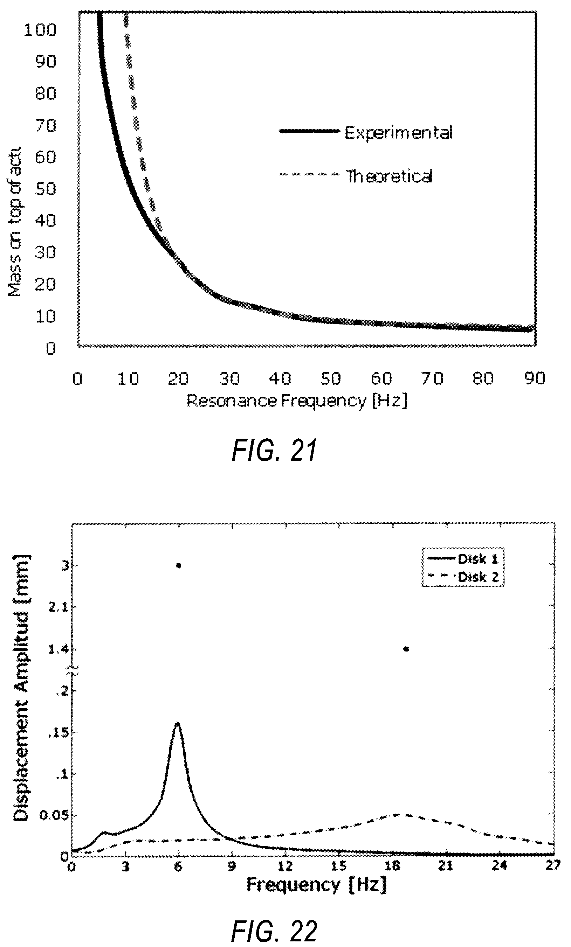

Figure 21 shows the comparison of experimental data to the theoretical estimation given an average k of 340 N/m. Based on the experimental curve, a mass at the tip of the piezoelectric actuator for $\omega d$ equal to 6 Hz and 19 Hz can be chosen. Consecutively, two materials with different densities (p) were chosen so that the outer radius and thickness of the mesh disks remained constant. The materials used were brass for the 6 Hz mesh disk and acrylonitrile butadiene styrene (ABS) for the 19 Hz mesh disk.

Each disk mesh was designed to have a different inner radius according to the needed R for the previously determined P. Considering the leak around the mesh disk, the FIMD was design to have a gap between the mesh disk and the surrounding wall of less than 0.5 mm. The measured resistance of such gap was 1 $cmH_2O/L/s$. After this, the inner radius and mesh's open area can be tuned to match the resistance used in Equation 5.

The piezoelectric actuator was driven at the maximum AC amplitude recommended by the manufacturer (50 Volts peak to peak). Pressure and flow were measured using a modified pneumotachometer with an extra pressure port (Figures 6A-8A) and a mesh resistance of 0.4 $cmH_2O/L/s$. Two ports, one on each side, were used for flow and the third for pressure. Both measurements are done using two highly symmetric 5 cm $H_2O$-range pressure transducers. The sensor data was acquired using a LABVIEW® acquisition card at 1000 Hz and the Zrs and signal to noise ratio (SNR) was calculated as follows: the signal at a given oscillation freq uency was taken as the peak at that frequency following the FFT, while the noise was estimated from the root mean squared average of the neighboring frequencies in a 0.5 Hz bandwidth on either side of the peak not containing any signal peaks. Thus for example for a 16 second recording, at 6 Hz the signal was the peak at 6 Hz and the noise was estimated from the root mean squared average of the values from 5.25 to 5.75 Hz and from 6.25 to 6.75 Hz. The SNR was then simply the signal divide by the noise.

Results:

The novel OS device was built according to the design and then tested using three test loads with a resistance to flow value of 1, 5, and 15 $cmH_2O/L/s$. The device has two interchangeable mesh disks that allowed it to apply pleasure waves at 6 Hz or 19 Hz. The body parts of the FIMD were made of ABS making it sturdy and light weighted (the total weight of the device is 495 grams).

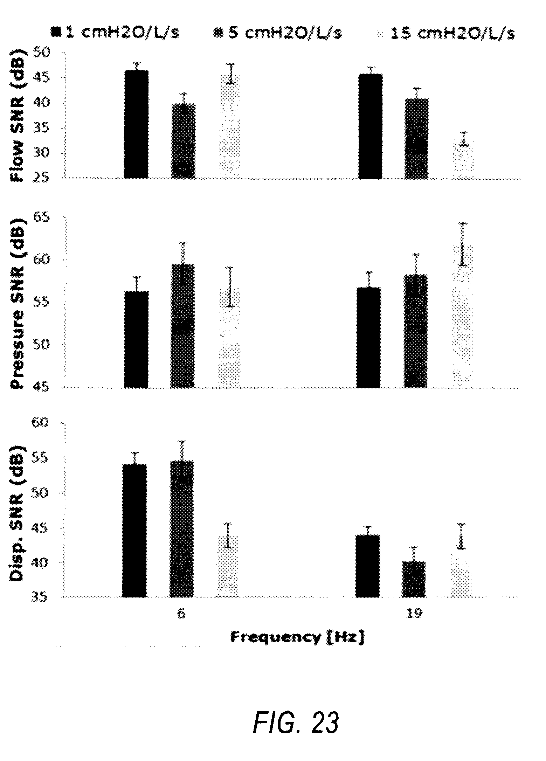

The traces in FIG. 20 show the frequency response of the mesh disk's displacement during a frequency sweep chirp input from 0 to 27 Hz separately with each mesh disk. Since this underestimates resonant performance due to the rate of the frequency sweep, the response at resonance indicated by the upper dots was measured together indicating adequate displacement performance at resonance. FIG. 23 shows the values of SNR in dB computed from 10 tests for flow, pressure and displacement, with the >30dB requirement exceeded for all test loads and both frequencies.

Conclusion:

This device is a proof of concept that an FIMD can be implemented in a compact, inexpensive, light-weighted and portable fashion with reliable performance. It represents a much simpler mechanical actuator design than any other approach presently known.

The FIMD takes advantage of the natural resonance of the actuator and thus requires very little power for operation; it could thus be battery operated. Given its characteristics and performance this device is particularly suited for easy assessment of respiratory mechanics for diagnosis and disease monitoring.

**Example 2. Multiple Piezoelectric Actuator FIMD**

The design of the multiple-actuator design was at least in part developed with the idea of increasing the stiffness of the piezoelectric actuator component of the FIMD. By doing so, the desired frequencies for the forced oscillations would be in the range of frequencies before the system reaches its resonance. Increasing the stiffness using multiple actuators would also increase the generated force, necessary to trade for amplification of the displacement using a lever mechanism. A minimum of 0.3 mm of amplitude at the tip of the actuators is expected before resonance frequency even with a large mass on top.

The longitude of the lever is dependent of the ratio between the distance from the input force to the pivot point and the distance from the output force to the pivot, and the area of the mesh-disk, a longer lever increases the displacement and a larger size of the mesh-disk decreases the displacement required. Using the graph in Figure 20 as reference, the size of the mesh-disk was selected to be close to 13000 $mm^2$ as well as the corresponding longitudes of input to pivot and output to pivot for the lever to generate an amplification factor of 13.

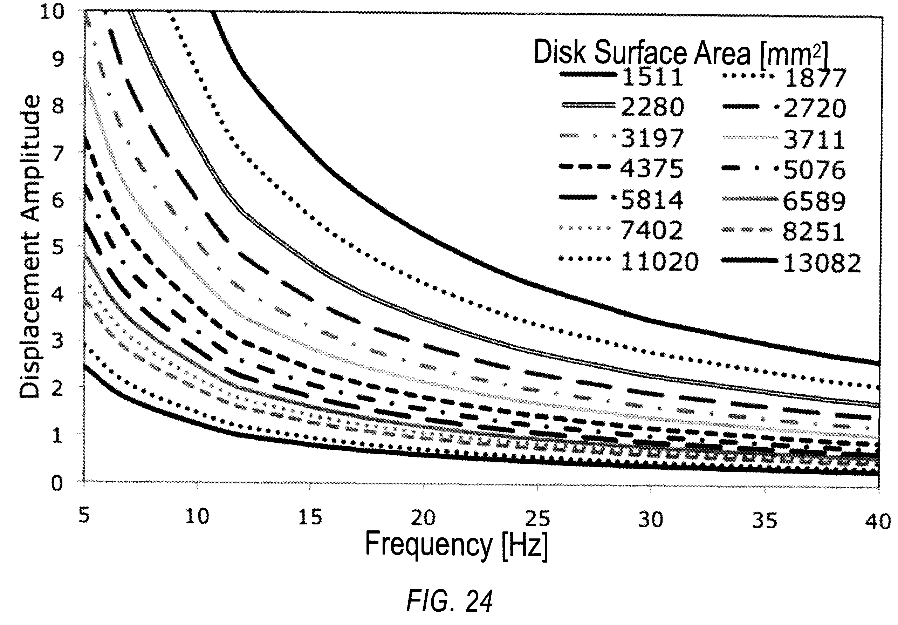

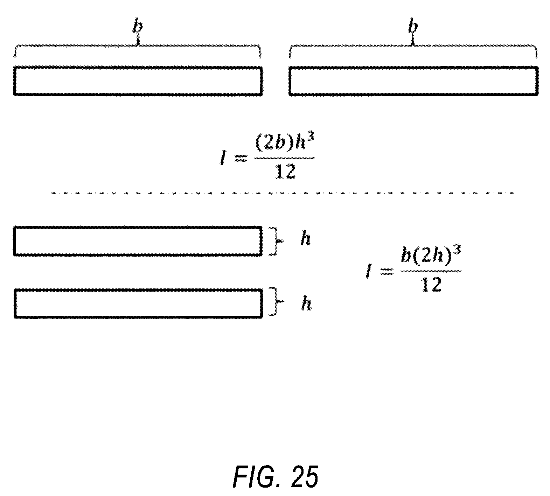

Static stiffness k of the system is increased based on the change of the equivalent (due to the different layers composing the beam) bending moment of inertia (see Figures 24 & 25) and by adjusting the active length of the beam L. Figure 25 is a top view of the tops of actuators in Figure 9A or 9B where the motion of the actuator tops would be towards the top and bottom of the page. Here b is the width of a piezo-actuator, h is the thickness and L is the length of actuator which is not shown as it descends below the page. Theoretically the stiffness k of a single actuator follows k = $3EI/L^3$, where E is the material property modulus of the piezoelectric material, and I is the moment of inertia. The equivalent stiffness of the multi-unit actuator can be calculated from the sum of the moments of actuators that are side by side as in the top panel of Fig. 25, and the sum of the moments of the actuators that are placed in front of each other as in the lower panel of Fig. 25. The higher the stiffness, the higher the resonant frequency $\omega_d$ as in Eq 7, and the greater the useful frequency range of the multi-actuator FIMD. The multi-actuator motor was designed then to be able to use 4, 6, 8, 10, or 12 actuators as shown herein, where the stiffness is increased by a maximum of 20.5 when using 12 actuators (See FIG.11, 11A-11C, and FIG.15).

Considering the leak around the mesh disk, the device was design to have a gap between the mesh disk and the surrounding wall of less than 1 mm (See FIG.18A). The gap can be increased compared to the single-actuator version due to expected imperfection in the alignment of the lever. The error in the positioning of the input force point is also amplified at the output, therefore, the need for more play around the mesh-disk. The decrease in leak resistance as a consequence of the gap can be compensated by decreasing the area of the wire mesh screen.

The drawings illustrate non-limiting illustrations of the parts and functions of the device.

The present invention has been described with reference to certain exemplary embodiments thereof. However, it will be readily apparent to those skilled in the art that it is possible to embody the invention in specific forms other than those of the exemplary embodiments described above. The exemplary embodiments are merely illustrative and should not be considered restrictive in any way. The scope of the invention is defined by the appended claims.

**Claims**

1. An actuator (10, 10', 10") connected to a structural ground (12, 12', 12") of a forced oscillation technique device (100, 200, 300, 400, 500, 600, 700, 800, 900, 1000), comprising:

   an electrical power source [16, 16',16"];
   a control device (17, 17', 17") connected to the electrical power source (16, 16', 16");
   a first portion (14a, 14a', 14a") including piezoelectric material connected to the electrical power source, and
   a second portion (14b, 14b', 14b") including non-piezoelectric, passive material connected to the first portion (14a, 14a', 14a"),

   wherein the second portion (14b, 14b', 14b") includes a ring member (24, 24', 24"),
   **characterized in that** the first portion (14a, 14a', 14a") includes at least one plate-shaped member (18, 18', 18"),
   wherein the ring member (24,24',24") is connected to and circumscribes a mesh screen (26,26',26").

2. The actuator (10,10', 10") according to claim 1, wherein the control device (17,17', 17") includes one or more of an amplifier and function generator for turning on, turning off or regulating an amount of power provided by the electrical power source (16, 16', 16") for causing oscillating movement ($X_+$, $X_-$) of a distal end (18b, 18b', 18b") of the at least one plate-shaped member (18, 18', 18") of the first portion (14a, 14a', 14a").

3. The actuator (10,10', 10") according to claim 1 or 2, wherein the second portion (14b, 14b', 14b") further includes an extension member coupler (20, 20', 20"), and
   an extension member (22, 22', 22"), wherein the extension member (22, 22', 22") is connected to the ring member (24, 24', 24"), wherein the extension member coupler (20, 20', 20") is connected to the distal end (18b, 18b', 18b") of the at least one plate-shaped member (18, 18', 18") of the first portion (14a, 14a', 14a").

4. The actuator (10,10',10") according to any one of claims 1-3, wherein a proximal end (18a, 18a', 18a") of the at least one plate-shaped member (18, 18', 18") is fixedly-connected to the structural ground (12, 12', 12") of the forced oscillation technique device (100, 200, 300, 400, 500, 600, 700, 800, 900, 1000).

5. The actuator (10) according to any one of claims 1-4, wherein the at least one plate-shaped member (18) includes one plate-shaped member thereby defining the actuator (10) as a single cantilever actuator, wherein particularly the oscillating movement ($X_+$, $X_-$) of the distal end (18b) of the one plate-shaped member (18) of the first portion (14a) causes a corresponding oscillating pivoting motion ($P_+$, $P_-$) of the second portion (14b) relative the structural ground (12), or the at least one plate-shaped member (18', 18") includes two or more plate-shaped members ($18_1'$-$18_n'$) thereby defining the actuator (10', 10") as a multi cantilever actuator, wherein particularly the oscillating movement ($H_+$, $X_-$) of the distal end (18b',18b") of the two or more plate-shaped members (18', 18") of the first portion (14a', 14a") translates into movement of the extension member coupler (20', 20") along an arcuate path (A), wherein movement of the extension member coupler (20', 20") along the arcuate path (A) translates into corresponding oscillating pivoting motion ($P_+$, $P_-$) of the extension member (22', 22"), ring member [24', 24"] and mesh screen (26',26") relative the structural ground (12', 12").

6. The actuator (10', 10") according to any one of claims 3-5, wherein the extension member coupler (20', 20") includes an elongated slot (20c', 20c") defined by opposing first and second end surfaces ($20c_1'$, $20c_2'$; $20c_1"$, $20c_2"$) that extend through a thickness of the extension member coupler (20', 20").

7. The actuator (10') according to any one of claims 3-6, wherein the extension member (22') extends from the structural ground (12') and through the elongated slot (20c') of the extension member coupler (20') such that that a distal end (22b') of the extension member (22') is arranged beyond a distal end (20b') of the extension member coupler (20'), wherein particularly the extension member (22') is indirectly connected to the two or more plate-shaped members (18') byway of a pin (25') extending entirely through the extension member coupler (20'), the elongated slot (20c') and a vertical slot (27') formed by a portion of a length ($22_L'$) of the extension member (22') that is substantially orthogonal to the elongated slot (20c') formed by the extension member coupler (20').

8. The actuator (10") according to any one of claims 3-7 further comprising
a pair of opposing pins (25") that partially extend into
a pivoting sleeve member (29") that is pivotally-arranged within the elongated slot (20c") of the extension member coupler (20") about a pivot axis (PP) that extends through the pair of opposing pins (25"), wherein the extension member (22") is slidably-coupled to the pivoting sleeve member (29").

9. A forced oscillation technique device (100, 200, 300, 400, 500, 600, 700, 800, 900, 1000), comprising:

a tube-shaped fluid-communicating member (102, 202, 302, 402, 502, 602, 702, 802, 902, 1002) defining a fluid-communicating passage (108, 208, 308, 408, 508, 608, 708, 808, 908, 1008);
a support member (104, 204, 304, 404, 504, 604, 704, 804, 904, 1004) supporting the tube-shaped fluid-communicating member (102, 202, 302, 402, 502, 602, 702, 802, 902, 1002), wherein the support member defines an actuator passage (104, 204, 304, 404, 504, 604, 704, 804, 904, 1004) that fluidly intersects the fluid-communicating passage (108, 208, 308, 408, 508, 608, 708, 808, 908, 1008) of the tube-shaped fluid-communicating member (102, 202, 302, 402, 502, 602, 702, 802, 902, 1002); and
an actuator (10, 10', 10") according to claim 1 connected to the support member (104, 204, 304, 404, 504, 604, 704, 804, 904,1004), wherein the actuator (10,10', 10") is disposed within the actuator passage (104, 204, 304, 404, 504, 604, 704,804, 904, 1004) and extends into the fluid-communicating passage (108, 208, 308, 408, 508, 608, 708, 808, 908, 1008),

wherein the at least one plate-shaped member (18, 18', 18") is movably ($X_+$ / $X_-$) disposed in the actuator passage (104, 204, 304, 404, 504,604, 704, 804, 904, 1004),
wherein the ring member (24, 24', 24") is movably ($P_+$ / $P_-$) disposed within the fluid-communicating passage (108, 208,308, 408, 508, 608, 708, 808, 908, 1008).

10. The forced oscillation technique device (100, 200, 300, 400, 500, 600, 700, 800, 900, 1000), according to claim 9, wherein an upstream opening (120, 220, 320, 420, 520, 620, 720, 820, 920,1020) of the fluid-communicating passage (108,208, 308, 408, 508, 608, 708, 808, 908, 008) is fluidly in communication with atmospheric pressure or with an anesthesia machine or mechanical ventilator (D).

11. The forced oscillation technique device (100, 200, 300, 400, 500, 600, 700, 800, 900, 1000), according to claim 9 or 10, wherein a downstream opening (122, 222, 322,422, 522, 622, 722, 822, 922, 1022) of the fluid-communicating passage (108, 208, 308, 408, 508, 608, 708, 808, 908,1008) is fluidly in communication with an oral human interface device (F, 1180), wherein particularly the oral human interface device (F) is a pneumotach, wherein more particularly the pneumotach (F) is communicatively coupled to the control device (17, 17', 17") of the actuator (10,10', 10"), or the oral human interface device (F) is an endotracheal tube (1180), wherein more particularly the endotracheal tube (1180) is communicatively coupled to the control device (17, 17', 17") of the actuator (10, 10', 10").

12. A method for determining the respiratory impedance (Zrs) of a subject, the method comprising:

a. providing a plurality of oscillations generated by a forced oscillation technique impedance measuring device (FIMD) to the airway of the subject, said device comprising:

i. an actuator (10, 10', 10") connected to a structural ground (12, 12', 12") of a forced oscillation technique (FOT) impedance measuring device (FIMD) (100, 200, 300, 400, 500, 600, 700, 800, 900, 1000), comprising:
ii. an electrical power source (16, 16', 16");
iii. a control device (17, 17', 17") connected to the electrical power source (16, 16', 16");
iv. a first portion (14a, 14a', 14a") including piezoelectric material connected to the electrical power source, and
v. a second portion (14b, 14b', 14b") including non-piezoelectic, passive material connected to the first portion (14a, 14a', 14a"), wherein the first portion (14a, 14a', 14a") includes
vi. at least one plate-shaped member (18, 8', 18"), wherein the second portion (14b, 14b', 14b") includes a ring member (24, 24', 24") connected to and circumscribing a mesh screen (26, 26', 26")

b. obtaining a pressure signal and a flow signal at each of a single, or a plurality of frequencies generated by said mesh screen;
c. collecting and processing said pressure signal and flow signal and
d. calculating an impedance (Zrs) of the subject from said pressure signal and said flow signal, wherein the frequency ranges from 4Hz to 34 Hz, and the frequency produced by said FIMD is matched to the damped resonance frequency ($\omega$d) of the actuator.

## Patentansprüche

1. Betätigungsvorrichtung (10, 10', 10"), die mit einem strukturellen Massepotential (12, 12', 12") einer Vorrichtung (100, 200, 300, 400, 500, 600, 700, 800, 900, 1000) zur Technik einer erzwungenen Schwingung verbunden ist, umfassend:

eine elektrische Leistungsquelle (16, 16', 16");
eine Steuervorrichtung (17, 17', 17"), die mit der elektrischen Leistungsquelle (16, 16', 16") verbunden ist;
einen ersten Abschnitt (14a, 14a', 14a"), der piezoelektrisches Material enthält, das mit der elektrischen Leistungsquelle verbunden ist, und
einen zweiten Abschnitt (14b, 14b', 14b"), der nicht-piezoelektrisches, passives Material enthält, das mit dem ersten Abschnitt (14a, 14a', 14a") verbunden ist,

wobei der zweite Abschnitt (14b, 14b', 14b") ein Ringelement (24, 24', 24") enthält,
**dadurch gekennzeichnet, dass** der erste Abschnitt (14a, 14a', 14a") mindestens ein plattenförmiges Element (18, 18', 18") enthält, wobei das Ringelement (24, 24', 24") mit einem Maschennetzschirm (26, 26', 26") verbunden ist und diesen umschreibt.

2. Betätigungsvorrichtung (10, 10', 10") nach Anspruch 1, wobei die Steuervorrichtung (17, 17', 17") einen oder mehrere eines Verstärkers und eines Funktionsgenerators enthält, um die Menge an Leistung, die von der elektrischen Leistungsquelle (16, 16', 16") bereitgestellt wird, anzuschalten, abzuschalten oder zu regulieren, um dadurch eine Schwingungsbewegung ($X_+$, $X_-$) eines distalen Endes (18b, 18b', 18b") des mindestens einen plattenförmigen Elements (18, 18', 18") des ersten Abschnitts (14a, 14a', 14a") zu veranlassen.

3. Betätigungsvorrichtung (10, 10', 10") nach Anspruch 1 oder 2, wobei der zweite Abschnitt (14b, 14b', 14b") ferner enthält:

ein Verbindungsstück eines Erweiterungselements (20, 20', 20"), und
ein Erweiterungselement (22, 22', 22"), wobei das Erweiterungselement (22, 22', 22") mit dem Ringelement (24, 24', 24") verbunden ist, wobei das Verbindungsstück des Erweiterungselements (20, 20', 20") mit dem distalen Ende (18b, 18b', 18b") des mindestens einen plattenförmigen Elements (18, 18', 18") des ersten Abschnitts (14a, 14a', 14a") verbunden ist.

4. Betätigungsvorrichtung (10, 10', 10") nach einem der Ansprüche 1 - 3, wobei ein proximales Ende (18a, 18a', 18a") des mindestens einen plattenförmigen Elements (18, 18', 18") fest mit dem strukturellen Massepotential (12, 12', 12") der Vorrichtung (100, 200, 300, 400, 500, 600, 700, 800, 900, 1000) zur Technik einer erzwungenen Schwingung verbunden ist.

**5.** Betätigungsvorrichtung (10) nach einem der Ansprüche 1 - 4, wobei das mindestens eine plattenförmige Element (18) ein plattenförmiges Element enthält, wodurch die Betätigungsvorrichtung (10) als eine Betätigungsvorrichtung mit einem einzelnen Ausleger definiert wird, wobei insbesondere die Schwingungsbewegung ($X_+$, $X_-$) des distalen Endes (18b) des einen plattenförmigen Elements (18) des ersten Abschnitts (14a) eine entsprechende Drehschwingungsbewegung ($P_+$, $P_-$) des zweiten Abschnitts (14b) relativ zu dem strukturellen Massepotential (12) veranlasst, oder wobei das mindestens eine plattenförmige Element (18', 18") zwei oder mehr plattenförmige Elemente ($18_1$' - $18_n$') enthält, wodurch die Betätigungsvorrichtung (10', 10") als eine Betätigungsvorrichtung mit mehreren Auslegern definiert wird, wobei insbesondere die Schwingungsbewegung ($X_+$, $X_-$) des distalen Endes (18b', 18b") der zwei oder mehr plattenförmigen Elemente (18', 18") des ersten Abschnitts (14a', 14a") in eine Bewegung des Verbindungsstücks des Erweiterungselements (20', 20") entlang eines Bogenpfads (A) übertragen wird, wobei die Bewegung des Verbindungsstücks des Erweiterungselements (20', 20") entlang des Bogenpfads (A) in eine entsprechende Drehschwingungsbewegung ($P_+$, $P_-$) des Erweiterungselements (22', 22"), des Ringelements (24', 24") und des Maschennetzschirms (26', 26") relativ zu dem strukturellen Massepotential (12', 12") übertragen wird.

**6.** Betätigungsvorrichtung (10', 10") nach einem der Ansprüche 3 - 5, wobei das Verbindungsstück des Erweiterungselements (20', 20") einen langgestreckten Schlitz (20c', 20c") enthält, der durch gegenüberliegende erste und zweite Endflächen ($20c_1$', $20c_2$', $20_{c1}$", $20c_2$"), die sich durch eine Dicke des Verbindungsstücks des Erweiterungselements (20', 20") erstrecken, definiert ist.

**7.** Betätigungsvorrichtung (10') nach einem der Ansprüche 3 - 6, wobei sich das Erweiterungselement (22') von dem strukturellen Massepotential (12') und durch den langgestreckten Schlitz (20c') des Verbindungsstücks des Erweiterungselements (20') derart erstreckt, dass ein distales Ende (22b') des Erweiterungselements (22') über einem distalen Ende (20b') des Verbindungsstücks des Erweiterungselements (20') angeordnet ist, wobei insbesondere das Erweiterungselement (22') indirekt mit den zwei oder mehr plattenförmigen Elementen (18') mittels eines Stiftes (25) verbunden ist, der sich gänzlich durch das Verbindungsstück des Erweiterungselements (20'), den langgestreckten Schlitz (20c') und durch einen vertikalen Schlitz (27') erstreckt, der durch einen Abschnitt einer Länge ($22_L$') des Erweiterungselements (22') gebildet ist und der im Wesentlichen senkrecht zu dem langgestreckten Schlitz (20c') ist, der von dem Verbindungsstück des Erweiterungselements (20') gebildet ist.

**8.** Betätigungsvorrichtung (10") nach einem der Ansprüche 3 - 7, ferner umfassend
ein Paar gegenüberliegender Stifte (25"), die sich teilweise in
ein drehbares Muffenelement (29") erstrecken, das innerhalb des langgestreckten Schlitzes (20c") des Verbindungsstücks des Erweiterungselements (20") um eine Schwenkachse (PP) schwenkbar angeordnet ist, die sich durch das Paar gegenüberliegender Stifte (25") erstreckt, wobei das Erweiterungselement (22") mit dem drehbaren Muffenelement (29") gleitend verbunden ist.

**9.** Vorrichtung (100, 200, 300, 400, 500, 600, 700, 800, 900, 1000) zur Technik einer erzwungenen Schwingung, umfassend:

ein rohrförmiges in Fluidkommunikation stehendes Element (102, 202, 302, 402, 502, 602, 702, 802, 902, 1002), das einen Durchlass (108, 208, 308, 408, 508, 608, 708, 808, 908, 1008) mit einer Fluidkommunikation definiert;
ein Trägerelement (104, 204, 304, 404, 504, 604, 704, 804, 904, 1004), das das rohrförmige in Fluidkommunikation stehende Element (102, 202, 302, 402, 502, 602, 702, 802, 902, 1002) trägt, wobei das Trägerelement einen Durchlass (104, 204, 304, 404, 504, 604, 704, 804, 904, 1004) einer Betätigungsvorrichtung definiert, der fluidtechnisch den Durchlass (108, 208, 308, 408, 508, 608, 708, 808, 908, 1008) mit einer Fluidkommunikation des rohrförmigen in Fluidkommunikation stehenden Elements (102, 202, 302, 402, 502, 602, 702, 802, 902, 1002) schneidet; und
eine Betätigungsvorrichtung (10, 10', 10") nach Anspruch 1, die mit dem Trägerelement (104, 204, 304, 404, 504, 604, 704, 804, 904, 1004) verbunden ist, wobei die Betätigungsvorrichtung (10, 10', 10") innerhalb des Durchlasses (104, 204, 304, 404, 504, 604, 704, 804, 904, 1004) der Betätigungsvorrichtung angeordnet ist und sich in den Durchlass (108, 208, 308, 408, 508, 608, 708, 808, 908, 1008) mit einer Fluidkommunikation erstreckt,

wobei das mindestens eine plattenförmige Element (18, 18', 18") in dem Durchlasses (104, 204, 304, 404, 504, 604, 704, 804, 904, 1004) der Betätigungsvorrichtung beweglich ($X_+$/$X_-$) angeordnet ist,
wobei das Ringelement (24, 24', 24") innerhalb des Durchlasses (108, 208, 308, 408, 508, 608, 708, 808, 908, 1008) mit einer Fluidkommunikation beweglich ($P_+$/$P_-$) angeordnet ist.

**10.** Vorrichtung (100, 200, 300, 400, 500, 600, 700, 800, 900, 1000) zur Technik einer erzwungenen Schwingung nach Anspruch 9, wobei eine Öffnung (120, 220, 320, 420, 520, 620, 720, 820, 920, 1020) stromaufwärts in dem Durchlass (108, 208, 308, 408, 508, 608, 708, 808, 908, 1008) mit einer Fluidkommunikation fluidtechnisch in Kommunikation mit dem atmosphärischen Druck oder mit einer Anästhesie-Maschine oder mit einem mechanischen Ventilator (D) steht.

**11.** Vorrichtung (100, 200, 300, 400, 500, 600, 700, 800, 900, 1000) zur Technik einer erzwungenen Schwingung nach Anspruch 9 oder 10, wobei eine Öffnung (122, 222, 322, 422, 522, 622, 722, 822, 922, 1022) stromabwärts in dem Durchlass (108, 208, 308, 408, 508, 608, 708, 808, 908, 1008) mit einer Fluidkommunikation fluidtechnisch in Kommunikation mit einer Vorrichtung (F, 1180) einer menschlichen Schnittstelle mit einem Mund steht, wobei vorzugsweise die Vorrichtung (F) der menschlichen Schnittstelle mit einem Mund ein Pneumotachogramm ist, wobei besonders vorzugsweise das Pneumotachogramm (F) gemeinsam mit der Steuervorrichtung (17, 17', 17") der Betätigungsvorrichtung (10, 10', 10") verbunden ist, oder die Vorrichtung (F) der menschlichen Schnittstelle mit einem Mund ein Endotrachealschlauch (1180) ist, wobei besonders vorzugsweise der Endotrachealschlauch (1180) gemeinsam mit der Steuervorrichtung (17, 17', 17") der Betätigungsvorrichtung (10, 10', 10") verbunden ist.

**12.** Verfahren zum Bestimmen der Atmungsimpedanz (Zrs) eines Subjekts, wobei das Verfahren umfasst:

    a. Bereitstellen mehrerer Schwingungen, die von einer Vorrichtung zur Impedanzmessung zur Technik einer erzwungenen Schwingung (FIMD) erzeugt werden, an den Luftweg der Person, wobei die Vorrichtung umfasst:

        i. eine Betätigungsvorrichtung (10, 10', 10"), die mit einem strukturellen Massepotential (12, 12', 12") einer Vorrichtung zur Impedanzmessung (FIMD) zur Technik einer erzwungenen Schwingung (FOT) (100, 200, 300, 400, 500, 600, 700, 800, 900, 1000) verbunden ist, umfassend:
        ii. eine elektrische Leistungsquelle (16, 16', 16");
        iii. eine Steuervorrichtung (17, 17', 17"), die mit der elektrischen Leistungsquelle (16, 16', 16") verbunden ist;
        iv. einen ersten Abschnitt (14a, 14a', 14a"), der piezoelektrisches Material enthält und mit der elektrischen Leistungsquelle verbunden ist, und
        v. einen zweiten Abschnitt (14b, 14b', 14b"), der nicht-piezoelektrisches, passives Material enthält und mit dem ersten Abschnitt (14a, 14a', 14a") verbunden ist, wobei der erste Abschnitt (14a, 14a', 14a") enthält
        vi. mindestens ein plattenförmiges Element (18, 18', 18"), wobei der zweite Abschnitt (14b, 14b', 14b") ein Ringelement (24, 24', 24") enthält, das mit einem Maschennetzschirm (26, 26', 26") verbunden ist und diesen umschreibt,

    b. Erhalten eines Drucksignals und eines Strömungssignals bei jeder einzelnen oder bei mehreren Frequenzen, die durch den Maschennetzschirm erzeugt werden;
    c. Sammeln und Verarbeiten des Drucksignals und des Strömungssignals und
    d. Berechnen der Impedanz (Zrs) der Person aus dem Drucksignal und dem Strömungssignal, wobei die Frequenz im Bereich von 4 Hz bis 34 Hz liegt, und wobei die Frequenz, die durch die FIMD erzeugt wird, mit der gedämpften Resonanzfrequenz ($\omega$d) der Betätigungsvorrichtung abgeglichen wird.

**Revendications**

**1.** Actionneur (10, 10', 10") relié à une mise à la terre de structure (12, 12', 12") d'un dispositif de technique d'oscillation forcée (100, 200, 300, 400, 500, 600, 700, 800, 900, 1000), comprenant :

    une source d'alimentation électrique (16, 16', 16") ;
    un dispositif de commande (17, 17', 17") relié à la source d'alimentation électrique (16, 16', 16") ;
    une première partie (14a, 14a', 14a") comprenant une matière piézoélectrique reliée à la source d'alimentation électrique, et
    une seconde partie (14b, 14b', 14b") comprenant une matière passive, non piézoélectrique reliée à la première partie (14a, 14a', 14a"),

    dans laquelle la seconde partie (14b, 14b', 14b") comprend un élément en anneau (24, 24', 24"),
    **caractérisé en ce que** la première partie (14a, 14a', 14a") comprend au moins un élément en forme de plaque (18, 18', 18"), dans lequel l'élément en anneau (24, 24', 24") est relié à un écran de maillage (26, 26', 26") et circonscrit à celui-ci.

**2.** Actionneur (10, 10', 10") selon la revendication 1, dans lequel le dispositif de commande (17, 17', 17") comprend un ou plusieurs d'un amplificateur et d'un générateur de fonctions pour allumer, éteindre ou réguler une quantité d'énergie fournie par la source d'alimentation électrique (16, 16', 16") pour provoquer un mouvement oscillatoire $(X_+, X_-)$ d'une extrémité distale (18b, 18b', 18b") de l'au moins un élément en forme de plaque (18, 18', 18") de la première partie (14a, 14a', 14a").

**3.** Actionneur (10, 10', 10") selon la revendication 1 ou 2, dans lequel la seconde partie (14b, 14b', 14b") comprend en outre
un coupleur d'élément de prolongement (20, 20', 20"), et
un élément de prolongement (22, 22', 22"), dans lequel l'élément de prolongement (22, 22', 22") est relié à l'élément en anneau (24, 24', 24"), dans lequel le coupleur d'élément de prolongement (20, 20', 20") est relié à l'extrémité distale (18b, 18b', 18b") de l'au moins un élément en forme de plaque (18, 18', 18") de la première partie (14a, 14a', 14a").

**4.** Actionneur (10, 10', 10") selon l'une quelconque des revendications 1 - 3, dans lequel une extrémité proximale (18a, 18a', 18a") de l'au moins un élément en forme de plaque (18, 18', 18") est reliée de manière fixée à la mise à la terre de structure (12, 12', 12") du dispositif de technique d'oscillation forcée (100, 200, 300, 400, 500, 600, 700, 800, 900, 1000).

**5.** Actionneur (10) selon l'une quelconque des revendications 1 - 4, dans lequel l'au moins un élément en forme de plaque (18) comprend un élément en forme de plaque définissant ainsi l'actionneur (10) comme un actionneur en porte-à-faux unique, dans lequel particulièrement le mouvement oscillatoire $(X_+, X_-)$ de l'extrémité distale (18b) de l'élément en forme de plaque (18) de la première partie (14a) provoque un mouvement de pivotement oscillatoire correspondant $(P_+, P_-)$ de la seconde partie (14b) par rapport à la mise à la terre de structure (12), ou l'au moins un élément en forme de plaque (18', 18") comprend deux ou plusieurs éléments en forme de plaque $(18_1' - 18_n")$ définissant ainsi l'actionneur (10', 10") comme un actionneur en porte-à-faux multiple, dans lequel particulièrement le mouvement oscillatoire $(X_+, X_-)$ de l'extrémité distale (18b', 18b") de deux ou plusieurs éléments en forme de plaque (18', 18") de la première partie (14a', 14") se traduit par un mouvement du coupleur d'élément de prolongement (20', 20") le long de la trajectoire arquée (A), dans lequel un mouvement du coupleur d'élément de prolongement (20', 20") le long de la trajectoire arquée (A) se traduit par un mouvement de pivotement oscillatoire correspondant $(P_+, P_-)$ de l'élément de prolongement (22', 22"), de l'élément d'anneau (24', 24") et de l'écran de maillage (26', 26") par rapport à la mise à la terre de structure (12', 12").

**6.** Actionneur (10', 10") selon l'une quelconque des revendications 3 - 5, dans lequel le coupleur d'élément de prolongement (20', 20") comprend une fente allongée (20c', 20c") définie en opposant des première et seconde surfaces d'extrémité $(20c_1', 20c_2' ; 20c_1", 20c_2")$ qui s'étendent à travers une épaisseur du coupleur d'élément de prolongement (20', 20").

**7.** Actionneur (10') selon l'une quelconque des revendications 3 - 6, dans lequel l'élément de prolongement (22') s'étend depuis la mise à la terre de structure (12') et à travers la fente allongée (20c') du coupleur d'élément de prolongement (20') de sorte qu'une extrémité distale (22b') de l'élément de prolongement (22') est agencée au-delà d'une extrémité distale (20b') du coupleur d'élément de prolongement (20'), dans lequel particulièrement l'élément de prolongement (22') est relié de manière indirecte aux deux ou plusieurs éléments en forme de plaque (18') au moyen d'une broche (25') s'étendant entièrement à travers le coupleur d'élément de prolongement (20'), la fente allongée (20c') et une fente verticale (27') formée par une partie d'une longueur $(22_L')$ de l'élément de prolongement (22') qui est substantiellement orthogonal à la fente allongée (20c') formée par le coupleur d'élément de prolongement (20').

**8.** Actionneur (10") selon l'une quelconque des revendications 3 - 7, comprenant en outre
une paire de broches opposées (25") qui s'étendent partiellement à l'intérieur de
un élément de manchon pivotant (29") qui est agencé de manière pivotante au sein de la fente allongée (20c") du coupleur d'élément de prolongement (20") autour d'un axe de pivotement (PP) qui s'étend à travers la paire de broches opposées (25"), dans lequel l'élément de prolongement (22") est couplée de manière coulissante à l'élément de manchon pivotant (29").

**9.** Dispositif de technique d'oscillation forcée (100, 200, 300, 400, 500, 600, 700, 800, 900, 1000), comprenant :

un élément en communication fluidique en forme de tube (102, 202, 302, 402, 502, 602, 702, 802, 902, 1002) définissant un passage en communication fluidique (108, 208, 308, 408, 508, 608, 708, 808, 908, 1008) ;
un élément de support (104, 204, 304, 404, 504, 604, 704, 804, 904, 1004) supportant l'élément en communication fluidique en forme de tube (102, 202, 302, 402, 502, 602, 702, 802, 902, 1002), dans lequel l'élément de support définit un passage d'actionneur (104, 204, 304, 404, 504, 604, 704, 804, 904, 1004) qui croise de manière fluidique le passage en communication fluidique (108, 208, 308, 408, 508, 608, 708, 808, 908, 1008) de l'élément en communication fluidique en forme de tube (102, 202, 302, 402, 502, 602, 702, 802, 902, 1002) ; et
un actionneur (10, 10', 10") selon la revendication 1 relié à l'élément de support (104, 204, 304, 404, 504, 604, 704, 804, 904, 1004), dans lequel l'actionneur (10, 10', 10") est disposé à l'intérieur du passage d'actionneur (104, 204, 304, 404, 504, 604, 704, 804, 904, 1004) et s'étend à l'intérieur du passage en communication fluidique (108, 208, 308, 408, 508, 608, 708, 808, 908, 1008),

dans lequel l'au moins un élément en forme de plaque (18, 18', 18") est disposé de manière amovible (X$_+$ / X$_-$) dans le passage d'actionneur (104, 204, 304, 404, 504, 604, 704, 804, 904, 1004),
dans lequel l'élément en anneau (24, 24', 24") est disposé de manière amovible (X$_+$ / X$_-$) à l'intérieur du passage en communication fluidique (108, 208, 308, 408, 508, 608, 708, 808, 908, 1008).

10. Dispositif de technique d'oscillation forcée (100, 200, 300, 400, 500, 600, 700, 800, 900, 1000), selon la revendication 9, dans lequel une ouverture en amont (120, 220, 320, 420, 520, 620, 720, 820, 920, 1020) du passage en communication fluidique (108, 208, 308, 408, 508, 608, 708, 808, 908, 1008) est en communication de manière fluidique avec la pression atmosphérique ou avec un appareil d'anesthésie ou un ventilateur mécanique (D).

11. Dispositif de technique d'oscillation forcée (100, 200, 300, 400, 500, 600, 700, 800, 900, 1000), selon la revendication 9 ou 10, dans lequel une ouverture en aval (122, 222, 322, 422, 522, 622, 722, 822, 922, 1022) du passage en communication fluidique (108, 208, 308, 408, 508, 608, 708, 808, 908, 1008) est en communication de manière fluidique avec un dispositif d'interface humaine par voie orale (F, 1180), dans lequel particulièrement le dispositif d'interface humaine par voie orale (F) est un pneumotachographe, dans lequel plus particulièrement le pneumotachographe (F) est couplé de manière communicative au dispositif de commande (17, 17', 17") de l'actionneur (10, 10', 10"), ou le dispositif d'interface humaine par voie orale (F) est un tube endotrachéal (1180), dans lequel plus particulièrement le tube endotrachéal (1180) est couplé de manière communicative au dispositif de commande (17, 17', 17") de l'actionneur (10, 10', 10").

12. Procédé pour déterminer l'impédance respiratoire (Zrs) d'un sujet, le procédé comprenant les étapes consistant à :

a. fournir une pluralité d'oscillations générées par un dispositif (FIMD) mesurant l'impédance utilisant une technique d'oscillation forcée aux voies respiratoires du sujet, ledit dispositif comprenant :

i. un actionneur (10, 10', 10") relié à une mise à la terre de structure (12, 12', 12") d'un dispositif (FIMD) mesurant l'impédance utilisant une technique d'oscillation forcée (FOT) (100, 200, 300, 400, 500, 600, 700, 800, 900, 1000), comprenant :
ii. une source d'alimentation électrique (16, 16', 16") ;
iii. un dispositif de commande (17, 17', 17") relié à la source d'alimentation électrique (16, 16', 16") ;
iv. une première partie (14a, 14a', 14a") comprenant une matière piézoélectrique reliée à la source d'alimentation électrique, et
v. une seconde partie (14b, 14b', 14b") comprenant une matière passive, non piézoélectrique reliée à la première partie (14a, 14a', 14a"), dans laquelle la première partie (14a, 14a', 14a") comprend
vi. au moins un élément en forme de plaque (18, 18', 18"), dans laquelle la seconde partie (14b, 14b', 14b") comprend un élément en anneau (24, 24', 24") relié à un écran de maillage (26, 26', 26") et circonscrit à celui-ci

b. obtenir un signal de pression et un signal d'écoulement à chacune d'une unique, ou d'une pluralité de fréquences générées par ledit écran de maillage ;
c. prélever et traiter lesdits signal de pression et signal d'écoulement et
d. calculer une impédance (Zrs) du sujet à partir dudit signal de pression et dudit signal d'écoulement, dans lequel la fréquence est comprise dans la gamme allant de 4 Hz à 34 Hz, et la fréquence produite par ledit FIMD est adaptée à la fréquence de résonance amortie (ωd) de l'actionneur.

**FIG. 1A**    **FIG. 1B**    **FIG. 1C**

EP 2 844 144 B1

*FIG. 2*

FIG. 3A

FIG. 3B

EP 2 844 144 B1

FIG. 4A

FIG. 4B

FIG. 4C

FIG. 5A

FIG. 5B

FIG. 5C

FIG. 6A

FIG. 7A

FIG. 7B

FIG. 8A

FIG. 7C

FIG. 6B

FIG. 8B

*FIG. 9B*

*FIG. 9A*

FIG. 10B

FIG. 10A

40

FIG. 11

FIG. 11A    FIG. 11B    FIG. 11C

600

10"

22"

20"

$18_1''-18_4''$

*FIG. 12*

700

10"

22"

20"

$18_1''-18_6''$

*FIG. 13*

800

10"

22"

20"

$18_1''-18_8''$

*FIG. 14*

900

10"

22"

20"

$18_1''-18_{10}''$

*FIG. 15*

EP 2 844 144 B1

43

FIG. 18A

FIG. 17A

FIG. 16A

FIG. 16B

FIG. 17B

FIG. 18B

EP 2 844 144 B1

FIG. 19A

FIG. 19B

FIG. 20

*FIG. 21*

*FIG. 22*

FIG. 23

*FIG. 24*

EP 2 844 144 B1

$$I = \frac{(2b)h^3}{12}$$

$$I = \frac{b(2h)^3}{12}$$

*FIG. 25*

FIG. 26

EP 2 844 144 B1

*FIG. 27*

FIG. 28

```
┌─────────────────────────────────────┐
│ 1 - Measure calibration impedances   │
│        Zc and store                   │
└─────────────────────────────────────┘
              │
              ▼
┌─────────────────────────────────────┐
│ 2 - Measure open impedance           │
│ (including any mouthpiece or filter)  │
│        Zo and store                   │
└─────────────────────────────────────┘
              │
              ▼
┌─────────────────────────────────────┐
│ 3 - Measure baseline subject          │
│ impedance Zm (t) over several cycles  │
│ and compensate to determine Zrs (t)   │
└─────────────────────────────────────┘
              │
              ▼
┌─────────────────────────────────────┐      ┌─────────────────────────────┐
│ 4 - Calculate Rsr, Xrs and variation  │- - ->│ 10 - Compare to standard     │
│        in Rrs and Xrs                 │      │ values and compute %         │
└─────────────────────────────────────┘      │ predicted to determine       │
              ┊                                │ if normal or abnormal        │
              ▼                                └─────────────────────────────┘
┌─────────────────────────────────────┐
│ 5- Administer bronchodilator          │
│    (or bronchoconstrictor)            │
└─────────────────────────────────────┘
              │
              ▼
┌─────────────────────────────────────┐
│ 6- Measure post drug subject          │
│ impedance Zmp and compensate          │
│     to determine Zrsp                 │
└─────────────────────────────────────┘
              │
              ▼
┌─────────────────────────────────────┐
│ 7 - Calculate Rsr, Xrs, and variation │
│        in Rrs and Xrs                 │
└─────────────────────────────────────┘
              │
              ▼
┌─────────────────────────────────────┐
│ 8 - Compare post-drug to pre drug     │
│ values of Rrs, Xrs, and variation in  │
│    Rrs and Xrs, respectively          │
└─────────────────────────────────────┘
              ┊
              ▼
┌─────────────────────────────────────┐
│ 9 - If increasing drug dose,          │
│ administer new dose and repeat        │
└─────────────────────────────────────┘
```

Optional

*FIG. 29*

20 - Measure calibration impedances Zc until coherence and signal to noise ratio at each frequency is acceptable and store

↓

21 - Measure open impedance Zo (including any mouthpiece or filter) until coherence and signal to noise ratio is acceptable and store

↓

22 - Measure baseline subject impedance Zm(t) and calculate Zm at each frequency until coherence and signal to noise ratio is acceptable and calculate Zm once per period of the perturbation waveform (typically 1 second)

↓

23 - Compensate Zm with Zo and Zc to compute baseline Zrs

↓

24 - Remove periods of Zrs for which coherence and/or signal to noise ratio was low

↓

25 - Calculate Rrs, Xrs and variation in Rrs and Xrs

31 - Compare to standard values and compute % predicted to determine if normal or abnormal

26 - Administer bronchodilator (or bronchoconstrictor)

↓

27 - Measure post drug subject impedance Zmp and compensate to determine Zrsp

↓

28 - Calculate Rrs, Xrs, and variation in Rrs and Xrs

↓

29 - Compare post-drug to baseline values of Rrs, Xrs, and variation in Rrs and Xrs, respectively

↓

30 - If increasing drug dose, administer new dose and repeat

*FIG. 30*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S60187799 A

- US 20110228968 A

**Non-patent literature cited in the description**

- **OOSTVEEN, E. et al.** The Forced Oscillation Technique In Clinical Practice: Methodology, Recommendations And Future Developments. *European Respiratory Journal,* 2003, vol. 22, 1026-1041
- **SCHUESSLER TF ; BATES JH.** A computer-controlled research ventilator for small animals: design and evaluation. *IEEE Trans Biomed Eng,* 1995, vol. 42, 860-866

- **DESAGER KN ; CAUBERGHS M ; VAN DE WOESTIJNE KP.** Two-point calibration procedure of the forced oscillation technique. *Med Biol Eng Comput.,* 1997
- Engineering vibration. **INMAN, D.** Upper Saddle River. Prentice Hall, 2001